# EUROPEAN PATENT APPLICATION

(11) **EP 3 831 882 A1**
(43) Date of publication of application: **09.06.2021**
(21) Application number: 19845535.4
(22) Date of filing: 31.07.2019
(51) Int. Cl.: C08L 61/04, C07C 39/14, C07D 311/78, C08G 8/00, C08G 8/28, G02B 1/04

(54) **OPTICAL COMPONENT-FORMING COMPOSITION, OPTICAL COMPONENT, COMPOUND, AND RESIN**

(30) Priority: 31.07.2018 JP 2018144516
(71) Applicant: MITSUBISHI GAS CHEMICAL COMPANY, INC., Chiyoda-ku Tokyo 100-8324 (JP)
(72) Inventor: OKADA, Yu, Hiratsuka-shi, Kanagawa 254-0016 (JP); TAKIGAWA, Tomoaki, Tokyo 100-8324 (JP); MAKINOSHIMA, Takashi, Hiratsuka-shi, Kanagawa 254-0016 (JP); ECHIGO, Masatoshi, Tokyo 100-8324 (JP)
(74) Representative: Müller-Boré & Partner Patentanwälte PartG mbB
(86) International application number: PCT/JP2019/030042
(87) International publication number: WO 2020/027206

(57) **Abstract**

Provided is a composition containing a polyphenol compound (B) and a solvent, in which the polyphenol compound (B) is at least one selected from a compound represented by the following formula (1) and a resin having a structure represented by the following formula (2) : 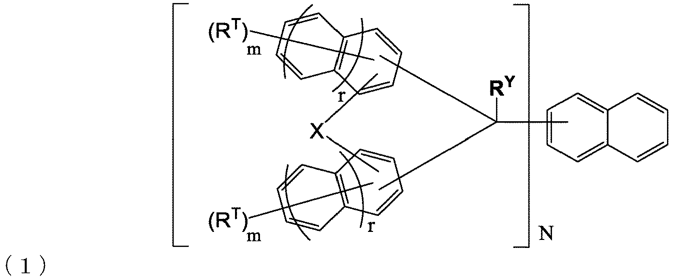 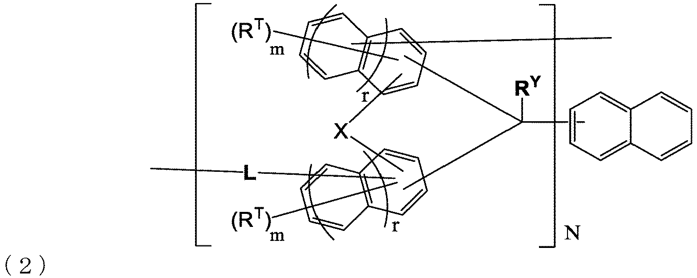 wherein R^{Y}, R^{T}, X, m, N, r, and L are as described in the description.

## Description

### Technical Field

The present invention relates to a compound and a resin having a specific structure, and to a composition for forming an optical component and an optical component containing the same.

### Background Art

In recent years, various compositions have been proposed as compositions for forming an optical component. Examples thereof include, for example, an acrylic resin, an epoxy-based resin or an anthracene derivative (see, for example, Patent Literatures 1 to 4 below). The present inventors have also developed useful polyphenol derivatives (see, for example, Patent Literature 5 below).

### Citation List

### Patent Literature

Patent Literature 1: Japanese Patent Laid-Open No. 2016-12061
Patent Literature 2: Japanese Patent Laid-Open No. 2015-174877
Patent Literature 3: Japanese Patent Laid-Open No. 2014-73986
Patent Literature 4: Japanese Patent Laid-Open No. 2010-138393
Patent Literature 5: International Publication No. WO 2018/052012

### Summary of Invention

### Technical Problem

However, although a large number of compositions for an optical component have heretofore been suggested, it is required to achieve all of storage stability, structure forming ability (film forming ability), heat resistance, transparency, and refractive index at high dimensions. In addition, there is a great demand for the development of a new material that satisfies the demand for a higher refractive index.

For this reason, development of a new material in which all of heat resistance, transparency, and refractive index are achieved at high dimensions is required.

An object of the present invention is to provide a composition for forming an optical component, an optical component, a compound, and a resin, which are capable of forming a cured product in which all of heat resistance, transparency, and refractive index are achieved at high dimensions.

### Solution to Problem

The present inventors have, as a result of devoted examinations to solve the above problems, found out that use of a compound or resin having a specific structure can solve the above problems, and reached the present invention.

More specifically, the present invention is as follows.
<1> A composition for forming an optical component, comprising a polyphenol compound (B) and a solvent,
   wherein the polyphenol compound (B) is at least one selected from a compound represented by the following formula (1) and a resin having a structure represented by the following formula (2): 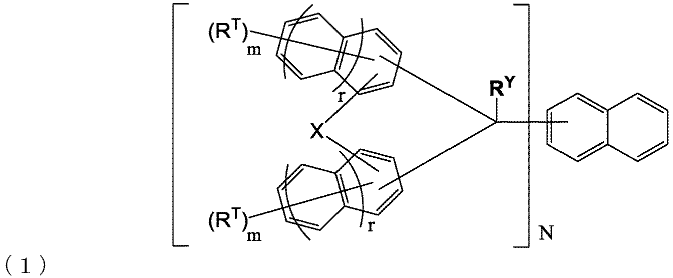 wherein
   R^{Y} is a hydrogen atom, an alkyl group having 1 to 30 carbon atoms and optionally having a substituent, or an aryl group having 6 to 40 carbon atoms and optionally having a substituent;
   each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
   X is an oxygen atom, a sulfur atom or not a crosslink;
   each m is independently an integer of 0 to 9, wherein at least one m is an integer of 1 to 9;
   N is an integer of 1 to 4, wherein when N is an integer of 2 or larger, N structural formulas within the parentheses [ ] are the same or different; and
   each r is independently an integer of 1 to 2, and 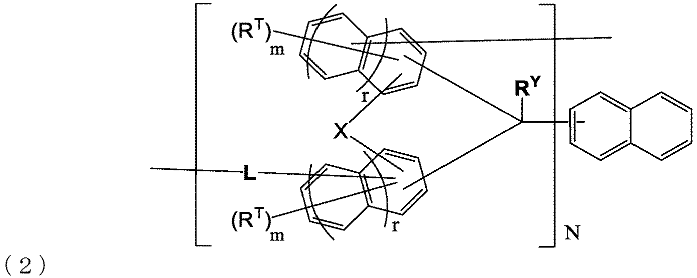 wherein L is an alkylene group having 1 to 30 carbon atoms and optionally having a substituent, an arylene group having 6 to 40 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 40 carbon atoms and optionally having a substituent;
   each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
   X is an oxygen atom, a sulfur atom or not a crosslink;
   each m is independently an integer of 0 to 9, wherein at least one m is an integer of 1 to 9;
   N is an integer of 1 to 4, wherein when N is an integer of 2 or larger, N structural formulas within the parentheses [ ] are the same or different; and
   each r is independently an integer of 1 to 2.
<2> The composition for forming an optical component according to the above <1>, wherein the polyphenol compound (B) is at least one selected from a compound represented by the following formula (1A) and a resin having a structure represented by the following formula (2A) : 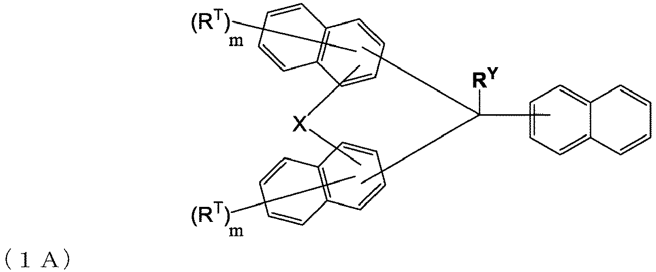 wherein R^{Y}, R^{T}, X, and m are as defined in the formula (1), and 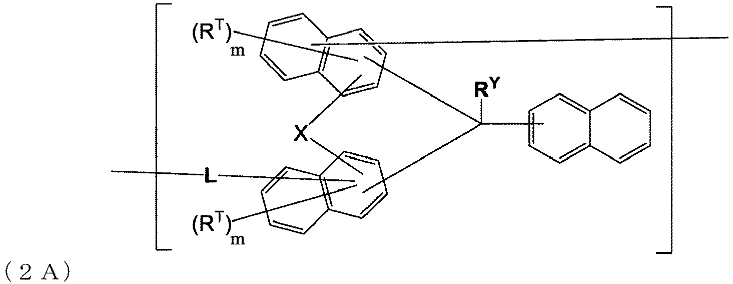 wherein L, R^{Y}, R^{T}, X, and m are as defined in the formula (2) .
<3> The composition for forming an optical component according to the above <1>, wherein the polyphenol compound (B) is at least one selected from a compound represented by the following formula (1B) or (1C) and a resin having a structure represented by the following formula (2B) or (2C): 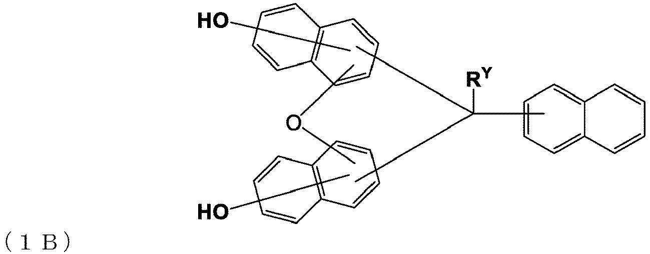 wherein R^{Y} is as defined in the formula (1), 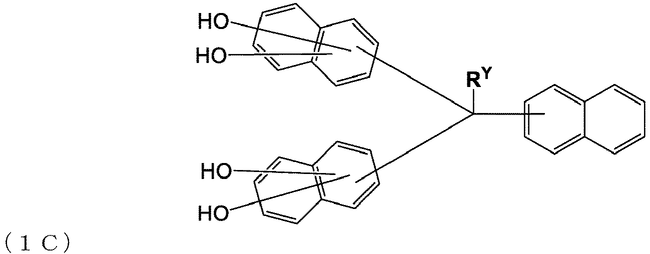 wherein R^{Y} is as defined in the formula (1), 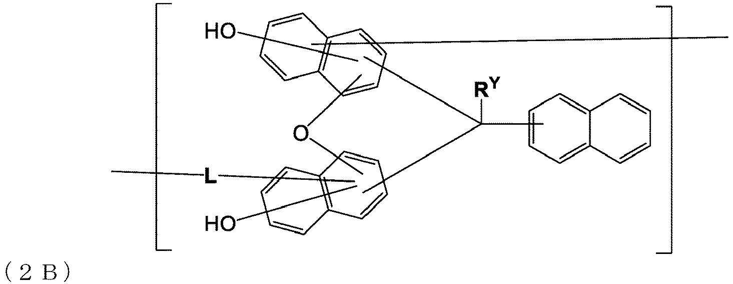 wherein L and R^{Y} are as defined in the formula (2), and 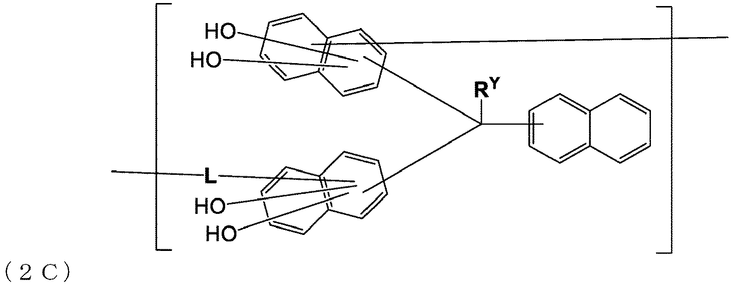 wherein L and R^{Y} are as defined in the formula (2).
<4> The composition for forming an optical component according to any one of the above <1> to <3>, further comprising an acid generating agent.
<5> The composition for forming an optical component according to any one of the above <1> to <4>, further comprising a crosslinking agent.
<6> A compound represented by the following formula (1) : 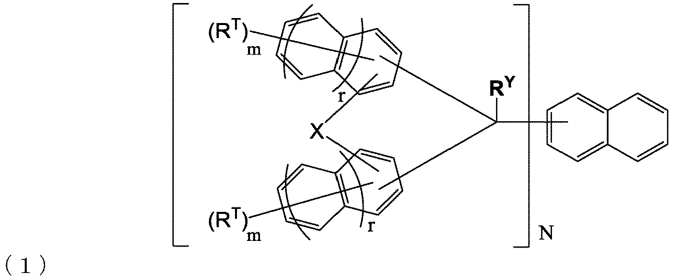 wherein
   R^{Y} is a hydrogen atom, an alkyl group having 1 to 30 carbon atoms and optionally having a substituent, or an aryl group having 6 to 40 carbon atoms and optionally having a substituent;
   each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
   X is an oxygen atom, a sulfur atom or not a crosslink;
   each m is independently an integer of 0 to 9, wherein at least one m is an integer of 1 to 9;
   N is an integer of 1 to 4, wherein when N is an integer of 2 or larger, N structural formulas within the parentheses [ ] are the same or different; and
   each r is independently an integer of 1 to 2.
<7> A resin having a structure represented by the following formula (2): 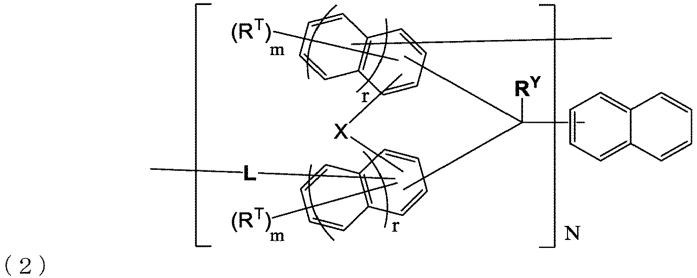
   wherein L is an alkylene group having 1 to 30 carbon atoms and optionally having a substituent, an arylene group having 6 to 40 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 40 carbon atoms and optionally having a substituent;
   each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
   X is an oxygen atom, a sulfur atom or not a crosslink;
   each m is independently an integer of 0 to 9, wherein at least one m is an integer of 1 to 9;
   N is an integer of 1 to 4, wherein when N is an integer of 2 or larger, N structural formulas within the parentheses [ ] are the same or different; and
   each r is independently an integer of 1 to 2.
<8> The compound according to the above <6>, wherein the compound is represented by the following formula (1A) : 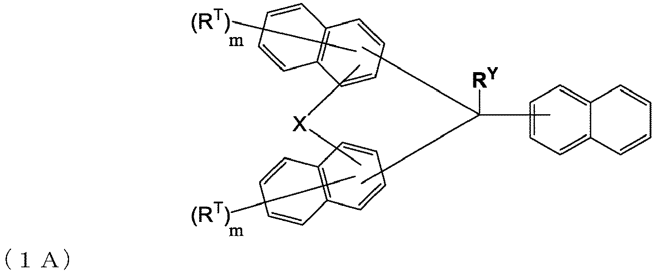 wherein R^{Y}, R^{T}, X, and m are as defined in the formula (1) .
<9> The resin according to the above <7>, wherein the resin has a structure represented by the following formula (2A): 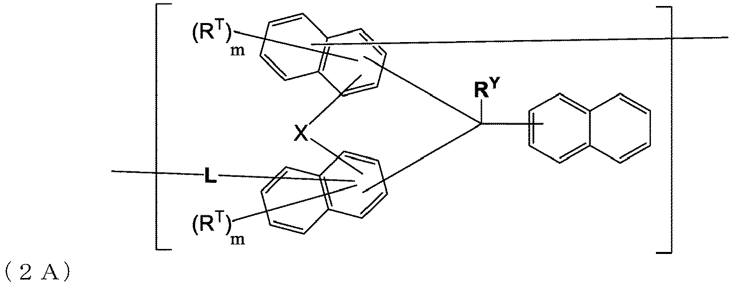 wherein L, R^{Y}, R^{T}, X, and m are as defined in the formula (2) .
<10> The compound according to the above <6>, wherein the compound is represented by the following formula (1B) or (1C): 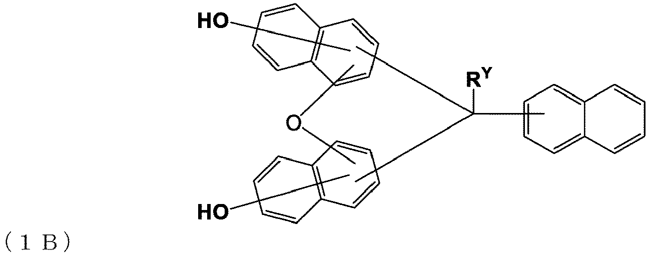 wherein R^{Y} is as defined in the formula (1), and 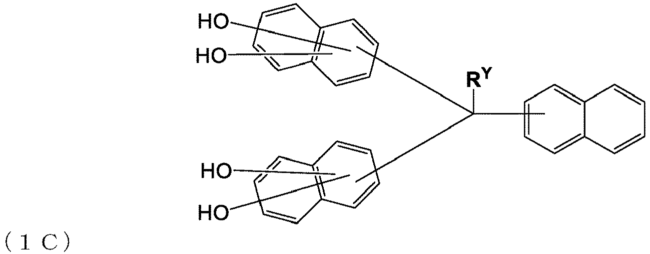 wherein R^{Y} is as defined in the formula (1).
<11> The resin according to the above <7>, wherein the resin has a structure represented by the following formula (2B) or (2C): 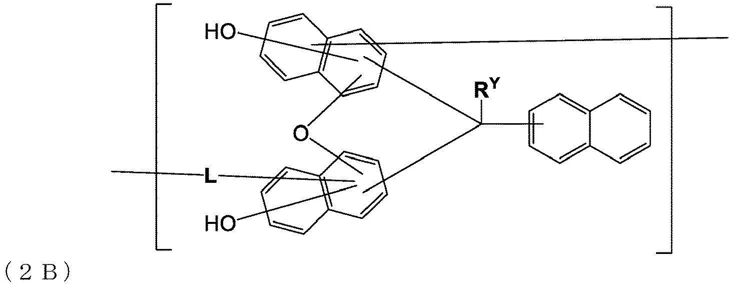 wherein L and R^{Y} are as defined in the formula (2), and 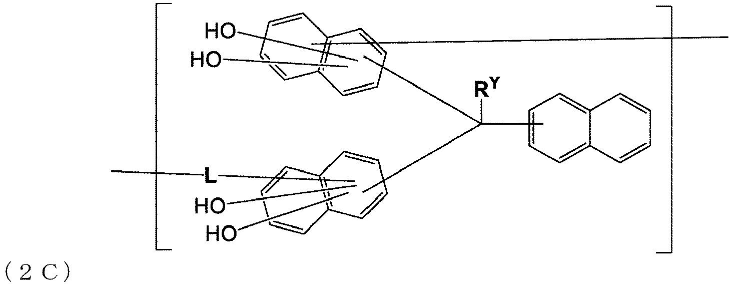 wherein L and R^{Y} are as defined in the formula (2).
<12> A method for producing the compound according to the above <6>, comprising the steps of replacing a hydrogen atom represented by R^{Y-0} of a compound represented by the following formula (1-0) with a hydroxy group; and replacing the hydroxy group with R^{Y-1} of a compound represented by the following formula (1-1): 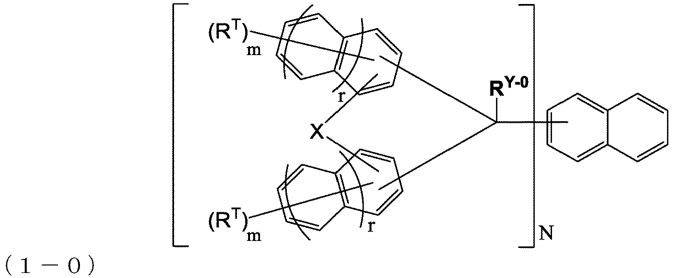 wherein
   R^{Y-0} is a hydrogen atom; and
   R^{T}, X, m, N, and r are as defined in the formula (1), and 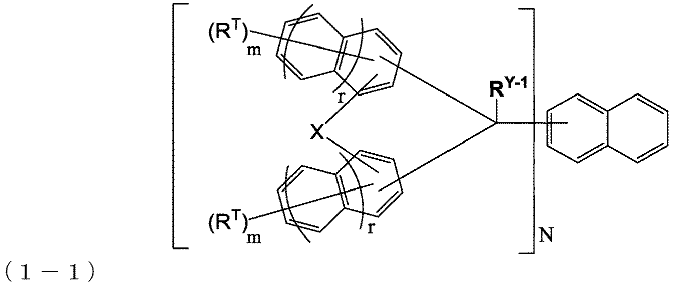 wherein
   R^{Y-1} is an alkyl group having 1 to 30 carbon atoms and optionally having a substituent, or an aryl group having 6 to 40 carbon atoms and optionally having a substituent;
   R^{T}, X, m, N, and r are as defined in the formula (1) .
<13> An optical component obtained from the composition for forming an optical component according to any one of the above <1> to <5>.

### Advantageous Effects of Invention

According to the present invention, it is possible to provide a composition for forming an optical component, an optical component, a compound, and a resin, which are capable of forming a cured product in which all of heat resistance, transparency, and refractive index are achieved at high dimensions.

### Description of Embodiments

Hereinafter, embodiments of the present invention will be described. The embodiments described below are given merely for illustrating the present invention. The present invention is not limited only by these embodiments. A compound and a resin used for a composition for forming an optical component of the present embodiment have high solubility in a safe solvent, and good heat resistance and etching resistance, as well as high refractive index, and is prevented from being stained by heat treatment in a wide range from a low temperature to a high temperature, and thus is useful as various composition for forming an optical components.

### <<Composition for forming an optical component>>

### (Polyphenol compound (B))

A composition of the present embodiment comprises a polyphenol compound (B) and a solvent, in which the polyphenol compound (B) is at least one selected from a compound represented by the following formula (1) and a resin having a structure represented by the following formula (2): 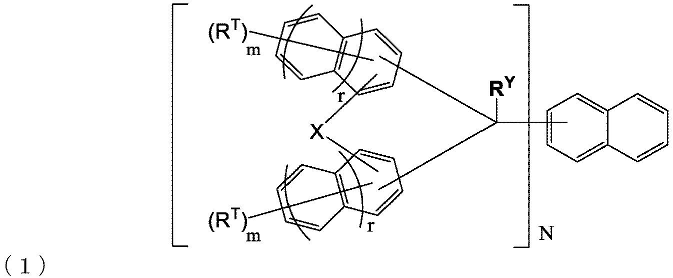 wherein
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2, and

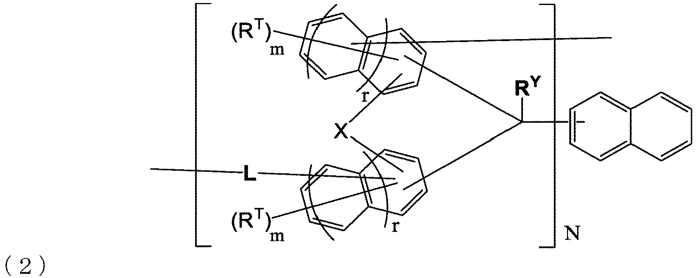

wherein L is an alkylene group having 1 to 30 carbon atoms and optionally having a substituent, an arylene group having 6 to 40 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2.

As for structural formulas described in the present specification, for example, when a line indicating a bond to C is in contact with a ring A and a ring B as described below, C is meant to be bonded to any one or both of the ring A and the ring B.

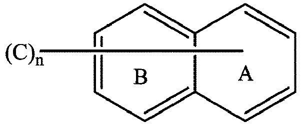

In the present specification, unless otherwise specified, the "alkyl group" includes linear, branched and cyclic alkyl groups.

Examples of the "alkyl group" having 1 to 30 carbon atoms for R^{Y} and R^{T} in each formula include a methyl group, an ethyl group, a n-propyl group, an i-propyl group, a n-butyl group, an i-butyl group, a t-butyl group, a cyclopropyl group, and a cyclobutyl group. From the viewpoint of solubility and heat resistance, the number of carbon atoms in the alkyl group is 1 to 30, preferably 1 to 20, more preferably 1 to 10, and particularly preferably 1 to 6.

Examples of the substituent for the alkyl group include a halogen atom, a nitro group, an amino group, a thiol group, a hydroxy group, and a group in which a hydrogen atom of a hydroxy group is replaced with a dissociation group or a crosslinking group.

Examples of the "aryl group" having 6 to 40 carbon atoms for R^{Y} and R^{T} in each formula include a phenyl group and a naphthyl group.

Examples of the substituent for the aryl group include a halogen atom, a nitro group, an amino group, a thiol group, a hydroxy group, a group in which a hydrogen atom of a hydroxy group is replaced with a dissociation group or a crosslinking group, and a phenyl group.

From the viewpoint of refractive index, the number of carbon atoms of the aryl group for R^{Y} in each formula is 6 to 40, preferably 6 to 24, more preferably 6 to 18, and particularly preferably 6 to 12.

From the viewpoint of refractive index, the number of carbon atoms of the aryl group for R^{T} in each formula is 6 to 40, preferably 6 to 24, more preferably 6 to 18, and particularly preferably 6 to 12.

Examples of the "alkenyl group" for R^{T} in each formula include a propenyl group and a butenyl group.

Examples of the substituent for the alkenyl group include a halogen atom, a nitro group, an amino group, a thiol group, a hydroxy group, and a group in which a hydrogen atom of a hydroxy group is replaced with a dissociation group or a crosslinking group.

From the viewpoint of solubility and heat resistance, the number of carbon atoms in the alkenyl group is 2 to 30, preferably 2 to 18, more preferably 2 to 12, and particularly preferably 2 to 6.

Examples of the "alkynyl group" for R^{T} in each formula include an alkynyl group having 2 to 30 carbon atoms such as an ethynyl group and a propagyl group. The alkynyl group optionally has a substituent. Examples of the substituent for the alkynyl group include a halogen atom, a nitro group, an amino group, a thiol group, a hydroxy group, and a group in which a hydrogen atom of a hydroxy group is replaced with a dissociation group or a crosslinking group.

From the viewpoint of solubility and heat resistance, the number of carbon atoms in the alkynyl group is 2 to 30, preferably 2 to 18, more preferably 2 to 12, and particularly preferably 2 to 6.

Examples of the "alkoxy group" for R^{T} in each formula include a methoxy group, an ethoxy group, a propoxy group, a cyclohexyloxy group, a phenoxy group, and a naphthoxy group.

Examples of the substituent for the alkoxy group include a halogen atom, a nitro group, an amino group, a thiol group, a hydroxy group, and a group in which a hydrogen atom of a hydroxy group is replaced with a dissociation group or a crosslinking group.

From the viewpoint of solubility and heat resistance, the number of carbon atoms in the alkoxy group is 1 to 30, preferably 1 to 18, more preferably 1 to 12, and particularly preferably 1 to 6.

Examples of the "halogen atom" for R^{T} in each formula include a fluorine atom, a chlorine atom, a bromine atom, and an iodine atom.

In the present embodiment, the "dissociation group" for R^{T} is a group that is dissociated by an acid, an alkali, or heat, and refers to a characteristic group that is cleaved in the presence of an acid to generate a functional group that alters solubility, such as an alkali soluble group. Examples of the alkali soluble group include a phenolic hydroxy group, a carboxyl group, a sulfonic acid group, and a hexafluoroisopropanol group. A phenolic hydroxy group and a carboxyl group are preferable, and a phenolic hydroxy group is particularly preferable. The dissociation group is preferably an acid dissociation group having the property of causing chained cleavage reaction in the presence of an acid, for achieving pattern formation with much higher sensitivity and higher resolution.

The acid dissociation group is not particularly limited, but can be arbitrarily selected for use from among, for example, those proposed in hydroxystyrene resins, (meth)acrylic acid resins, and the like for use in chemically amplified resist compositions for KrF or ArF. Specific examples of the acid dissociation group include those described in International Publication No. WO 2016/158168.

In the present specification, the term "crosslinking group" for R^{T} refers to a group that crosslinks by an acid, an alkali, light or heat, and crosslinks in the presence of a catalyst or without a catalyst. Examples of the crosslinking group include, but not particularly limited to, a group having an allyl group, a group having a (meth)acryloyl group, a group having an epoxy (meth)acryloyl group, a group having a urethane (meth)acryloyl group, a group having a hydroxy group, a group having a glycidyl group, a group having a vinyl containing phenylmethyl group, a group having a styrene group, a group having an alkynyl group, a group having a carbon-carbon double bond, a group having a carbon-carbon triple bond, and a group containing these groups.

Examples of the "group having an allyl group" include, but not particularly limited to, a group represented by the following formula (X-1).

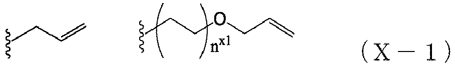

In the formula (X-1), n^{X1} is an integer of 1 to 5.

Examples of the group having a (meth)acryloyl group include, but not particularly limited to, a group represented by the following formula (X-2).

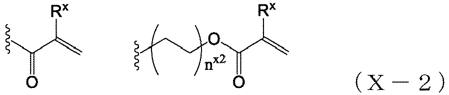

In the formula (X-2), n^{X2} is an integer of 1 to 5, and R^{X} is a hydrogen atom or a methyl group.

Examples of the group having "an epoxy (meth)acryloyl group" include, but not particularly limited to, a group represented by the following formula (X-3). Here, the epoxy (meth)acryloyl group refers to a group generated through a reaction between an epoxy (meth)acrylate and a hydroxy group.

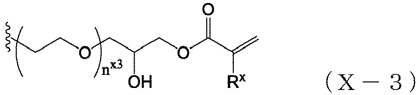

In the formula (X-3), n^{x3} is an integer of 0 to 5, and R^{X} is a hydrogen atom or a methyl group.

Examples of the group having "a urethane (meth)acryloyl group" include, but not particularly limited to, a group represented by the following formula (X-4) .

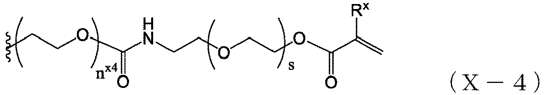

In the formula (X-4), n^{X4} is an integer of 0 to 5; s is an integer of 0 to 3; and R^{X} is a hydrogen atom or a methyl group.

Examples of the "group having a hydroxy group" include, but not particularly limited to, a group represented by the following formula (X-5).

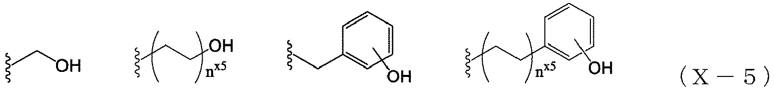

In the formula (X-5), n^{X5} is an integer of 1 to 5.

Examples of the "group having a glycidyl group" include, but not particularly limited to, a group represented by the following formula (X-6).

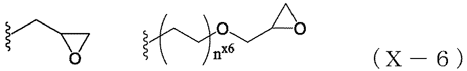

In the formula (X-6), n^{X6} is an integer of 1 to 5.

Examples of the group having "a vinyl containing phenylmethyl group" include, but not particularly limited to, a group represented by the following formula (X-7).

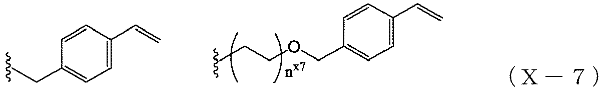

In the formula (X-7), n^{X7} is an integer of 1 to 5.

Examples of the "group having a styrene group" include, but not particularly limited to, a group represented by the following formula (X-8).

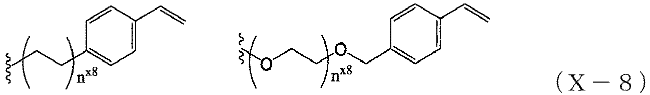

In the formula (X-8), n^{X8} is an integer of 1 to 5.

Examples of the group having various alkynyl groups include, but not particularly limited to, a group represented by the following formula (X-9). 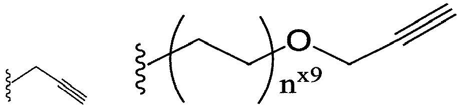 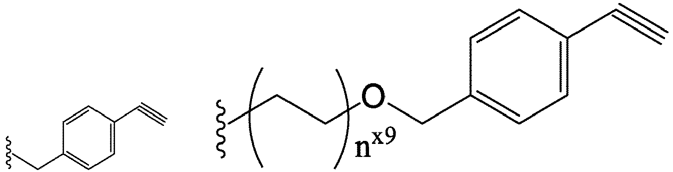 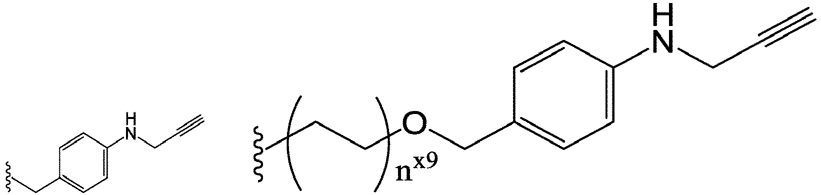 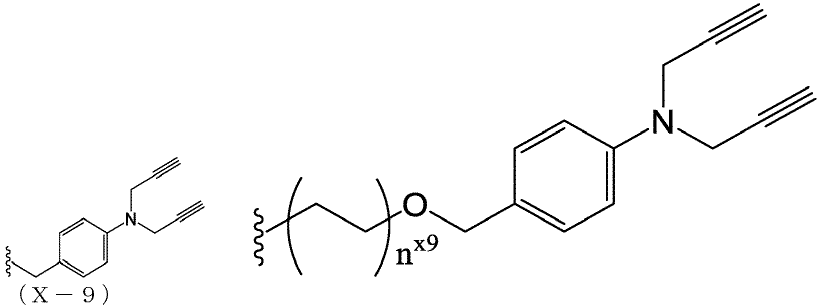 In the formula (X-9), n^{X9} is an integer of 1 to 5.

Examples of the carbon-carbon double bond containing group include a (meth)acryloyl group, a substituted or unsubstituted vinyl phenyl group, and a group represented by the following formula (X-10-1).

In addition, examples of the carbon-carbon triple bond containing group include a substituted or unsubstituted ethynyl group, a substituted or unsubstituted propargyl group, and a group represented by the following formula (X-10-2) or (X-10-3).

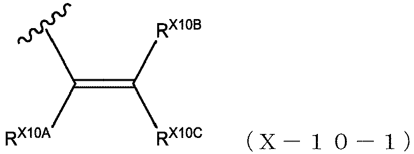

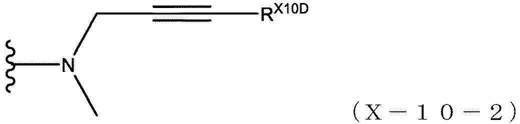

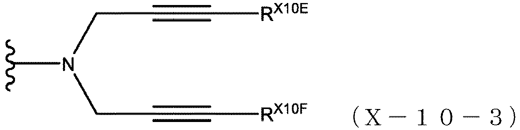

In the above formula (X-10-1), R^{X10A}, R^{X10B} and R^{X10C} are each independently a hydrogen atom or a monovalent hydrocarbon group having 1 to 20 carbon atoms. In the above formulas (X-10-2) and (X-10-3), R^{X10D}, R^{X10E} and R^{X10F} are each independently a hydrogen atom or a monovalent hydrocarbon group having 1 to 20 carbon atoms.

Among the above, from the viewpoint of ultraviolet curability, the crosslinking group is preferably a group having a (meth)acryloyl group, an epoxy (meth)acryloyl group, a urethane (meth)acryloyl group, a glycidyl group, or a styrene group, more preferably a group having a (meth)acryloyl group, an epoxy (meth)acryloyl group or a urethane (meth)acryloyl group, and still more preferably a group having a (meth)acryloyl group.

In the formulas (1) and (2), X is an oxygen atom, a sulfur atom, or not a crosslink. X is preferably an oxygen atom or a sulfur atom and more preferably an oxygen atom, because there is a tendency to exhibit high heat resistance. Preferably, X is not a crosslink from the viewpoint of solubility. Each m is independently an integer of 0 to 9, and at least one m is an integer of 1 to 9. N is an integer of 1 to 4. When N is an integer of 2 or larger, N structural formulas within the parentheses [ ] may be the same or different.

In the formulas (1) and (2), a site represented by the naphthalene structure represents a monocyclic structure when r is 0, a bicyclic structure when r is 1, and a tricyclic structure when r is 2. Each r is independently an integer of 0 to 2. The numeric range of m mentioned above depends on the ring structure defined by r.

A resin having a structure represented by the formula (2) is a resin having a unit structure derived from the compound represented by the formula (1). For example, the resin can be synthesized by reacting the compound represented by the above formula (1) with a crosslinking compound.

In the formula (2), L is an alkylene group having 1 to 30 carbon atoms optionally having a substituent, an arylene group having 6 to 40 carbon atoms optionally having a substituent, an alkoxylene group having 1 to 30 carbon atoms optionally having a substituent or a single bond. The alkylene group, the arylene group, and the alkoxylene group each optionally contain an ether bond, a ketone bond, or an ester bond. Each of the alkylene group and the alkoxylene group may be a linear, branched, or a cyclic group.

The polyphenol compound (B) is preferably selected from a compound represented by the following formula (1A) and a resin having a structure represented by the following formula (2A) from the viewpoint of high refractive index. The reason why the high refractive index is achieved is not clear, but it is presumed to be due to the high aromatic density in the composition.

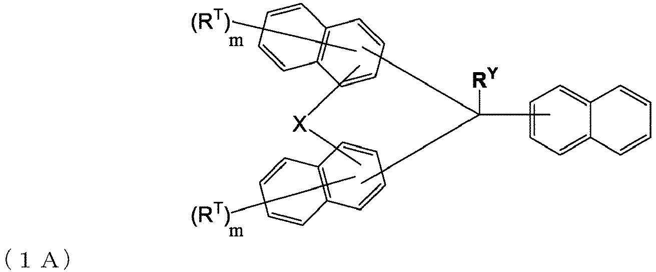

In the formula (1A), R^{Y}, R^{T}, X, and m are as defined in the formula (1).

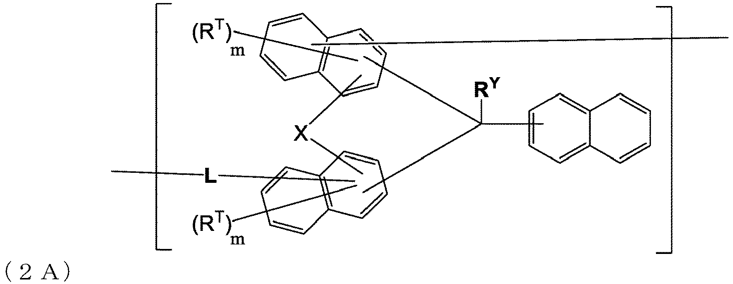

In the formula (2A), L, R^{Y}, R^{T}, X, and m are as defined in the formula (2).

The polyphenol compound (B) is preferably selected from a compound represented by the following formula (1B) or (1C) and a resin having a structure represented by the following formula (2B) or (2C) from viewpoint of structure forming ability (film forming ability).

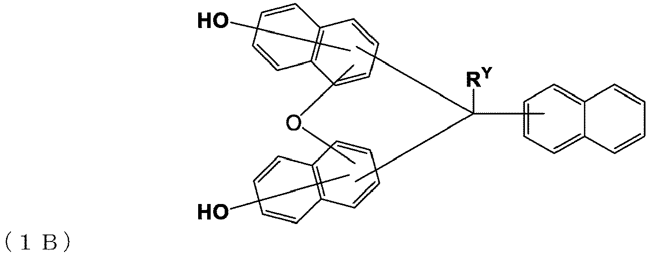

In the formula (1B), R^{Y} is as defined in the formula (1) .

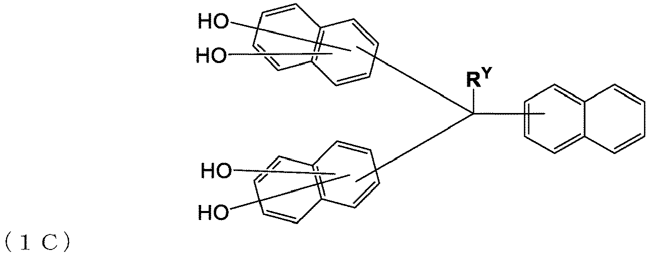

In the formula (1C), R^{Y} is as defined in the formula (1) .

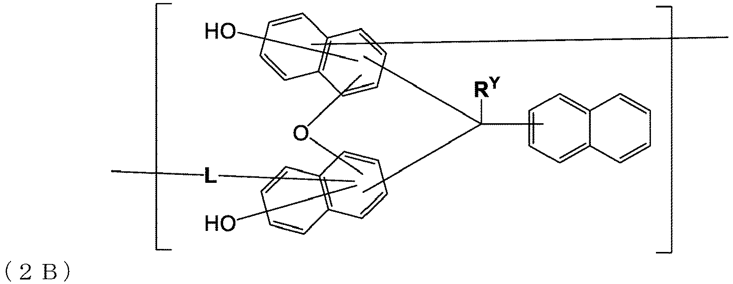

In the formula (2B), L and R^{Y} are as defined in the formula (2).

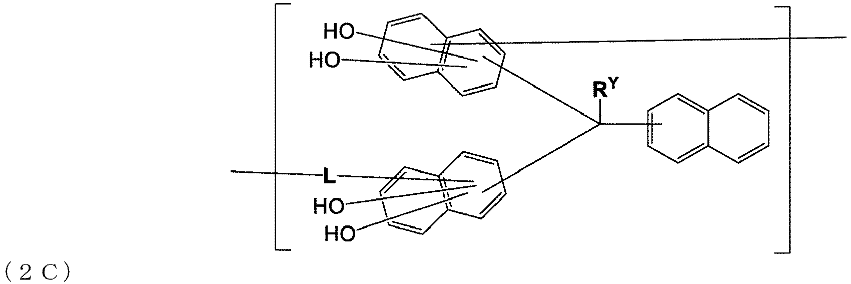

In the formula (2C), L and R^{Y} are as defined in the formula (2).

The compound represented by the formula (1) and the resin represented by the formula (2) have high refractive index, and are prevented from being stained by heat treatment in a wide range from a low temperature to a high temperature. Therefore, they can be suitably used for compositions for forming various optical components to thereby exhibit the effect. The optical component is used in the form of a film or a sheet and is also useful as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, a photosensitive optical waveguide, a liquid crystal display, an organic electroluminescent (EL) display, an optical semiconductor (LED) element, a solid state image sensing element, an organic thin film solar cell, a dye sensitized solar cell, and an organic thin film transistor (TFT). It can be particularly suitably utilized as an embedded film and a smoothed film on a photodiode, a smoothed film in front of or behind a color filter, a microlens, and a smoothed film and a conformal film on a microlens, all of which are parts of a solid state image sensing element, to which high refractive index is demanded.

Specific examples of the compound represented by the above formula (1) include compounds represented by the following formulas. However, the compound represented by the above formula (1) is not limited to the compounds represented by the following formulas.

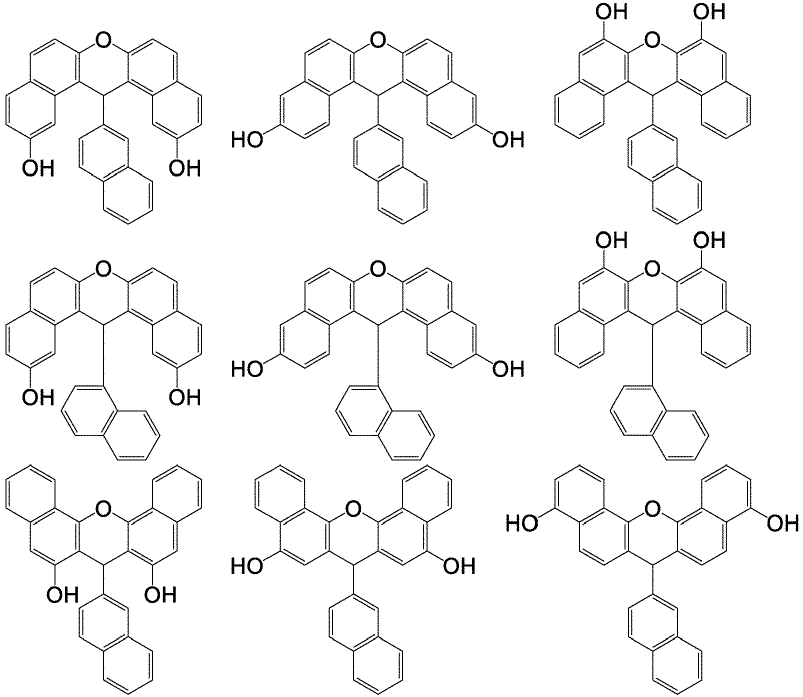

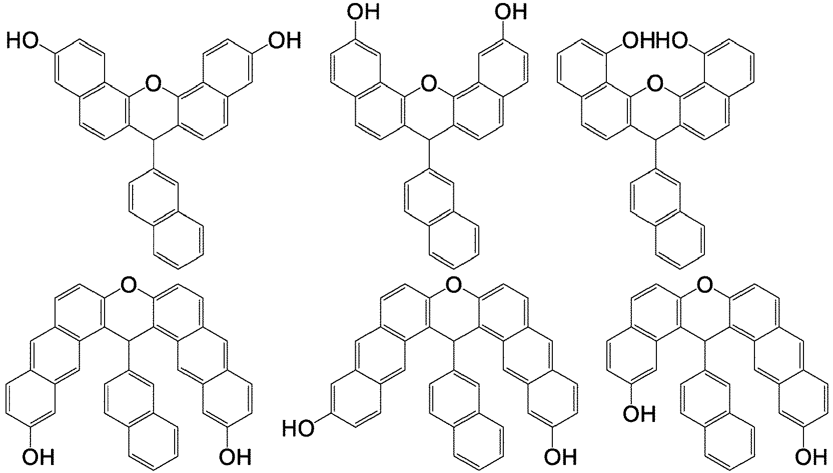

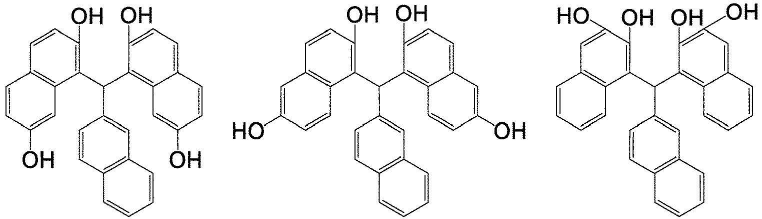

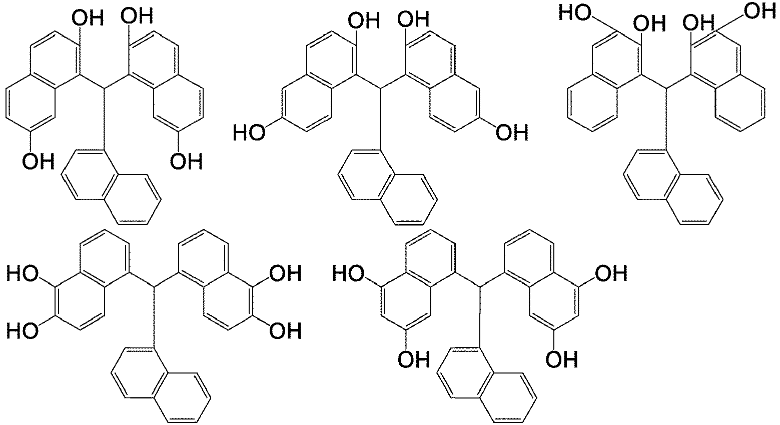

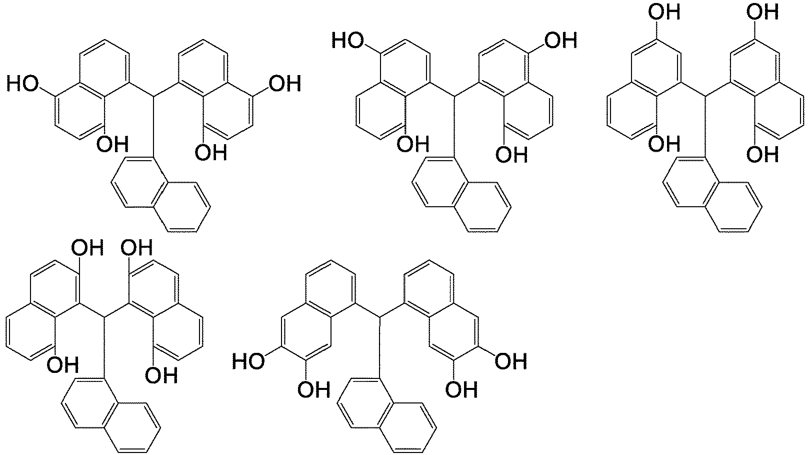

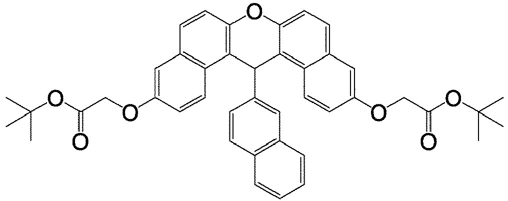

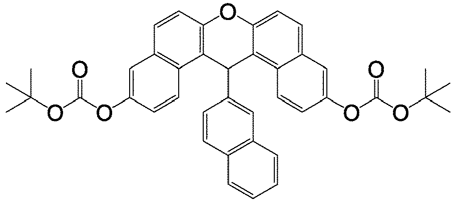

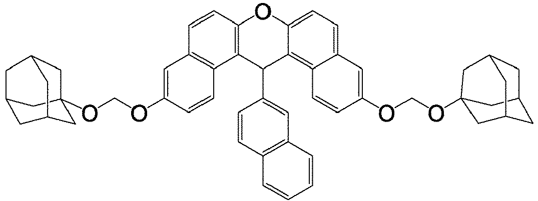

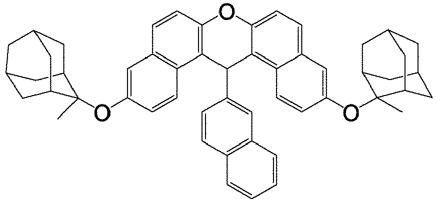

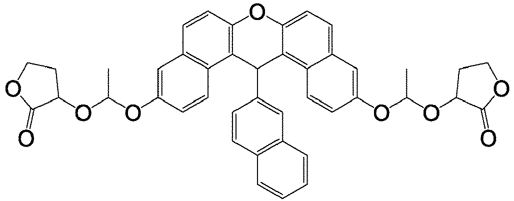

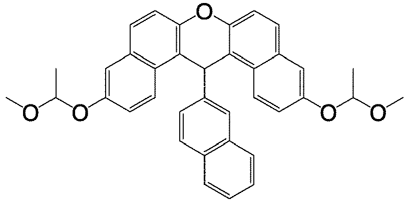

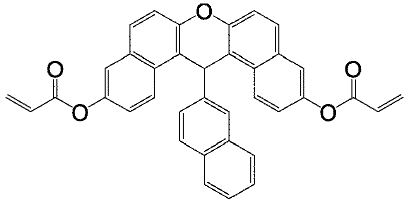

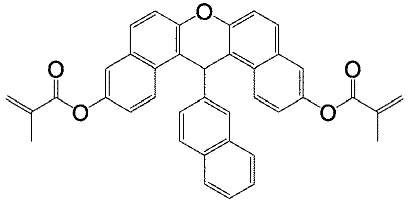

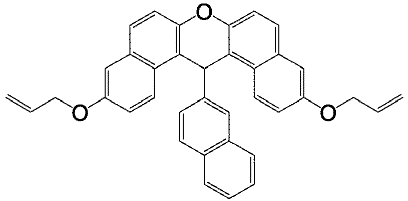

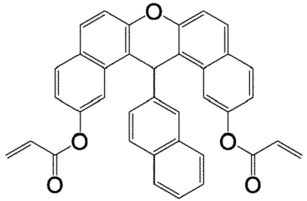

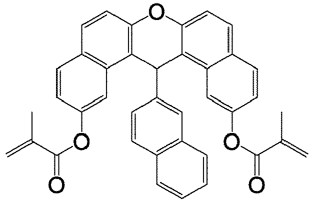

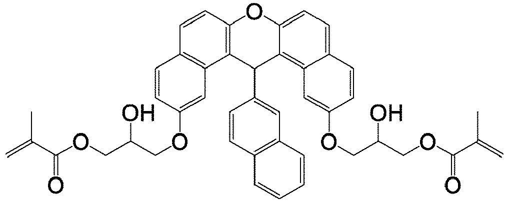

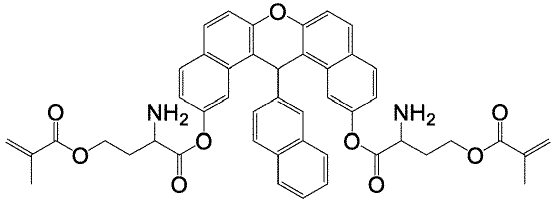

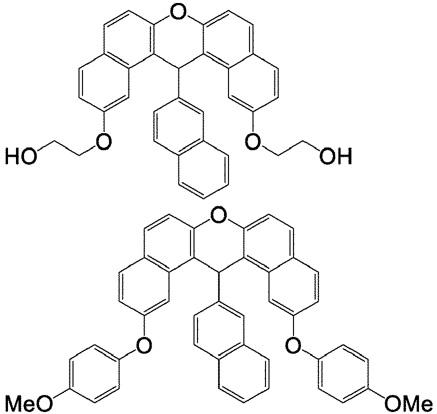

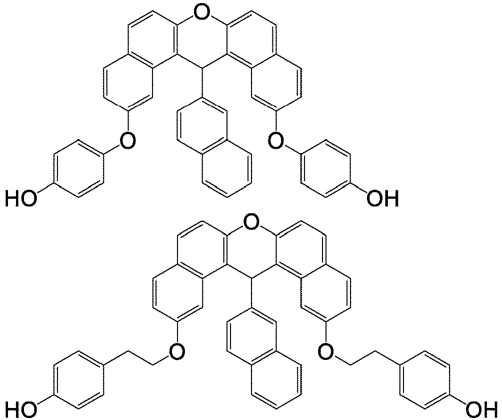

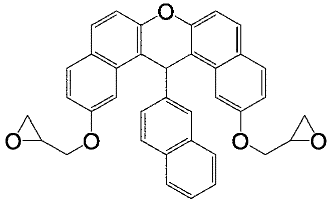

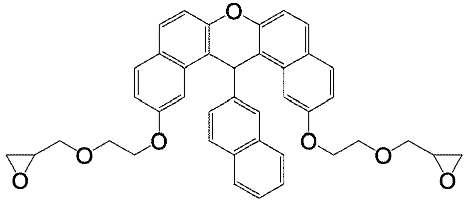

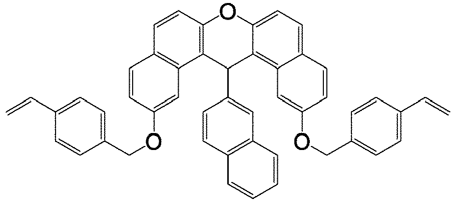

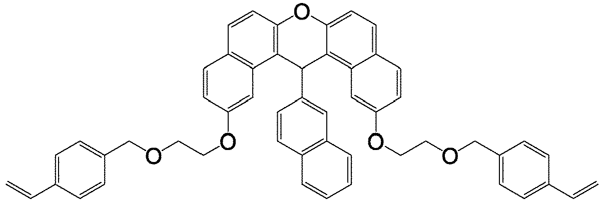

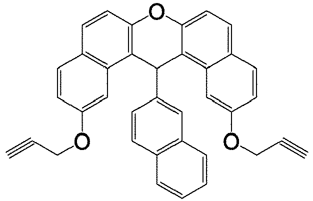

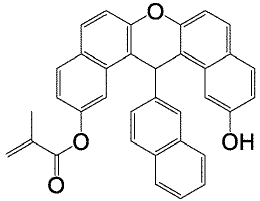

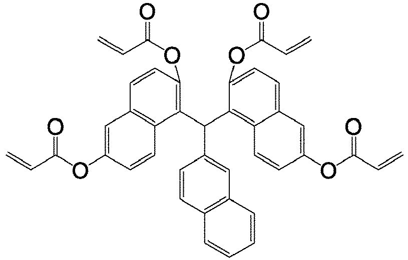

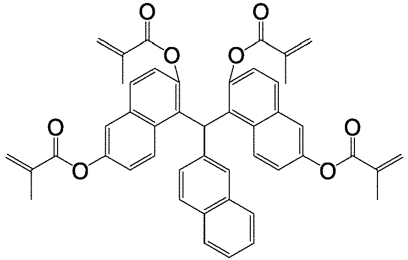

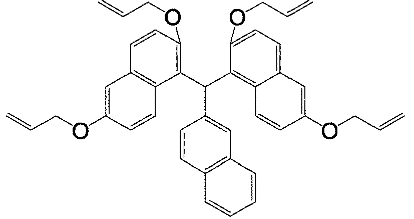

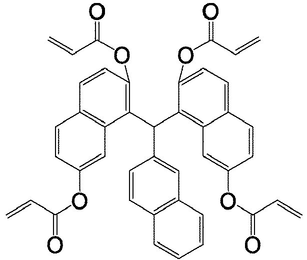

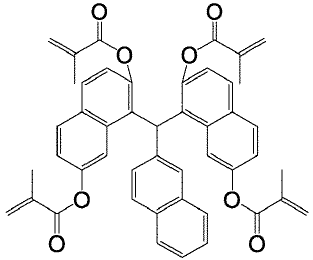

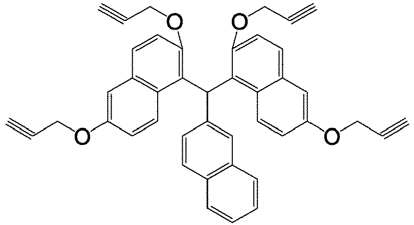

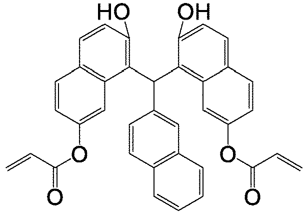

### [Method for producing compound represented by formula (1) ]

The compound represented by the formula (1) used in the present embodiment can be arbitrarily synthesized by the application of a publicly known approach, and the synthesis approach is not particularly limited. For example, it can be obtained by, for example, (i) subjecting a dihydroxynaphthalene or a dihydroxyanthracene and a corresponding aldehyde or ketone to polycondensation reaction in the presence of an acid catalyst.

Examples of (i) the method of subjecting a dihydroxynaphthalene or a dihydroxyanthracene and a corresponding aldehyde or ketone to polycondensation reaction in the presence of an acid catalyst include (a) a method of carrying out the polycondensation reaction in an organic solvent, (b) a method of carrying out the polycondensation reaction in an aqueous solvent, and (c) a method of carrying out the polycondensation reaction with no solvent.

In (a) the method of subjecting a dihydroxynaphthalene or a dihydroxyanthracene and a corresponding aldehyde or ketone to polycondensation reaction in the presence of an acid catalyst in an organic solvent, the compound represented by the above formula (1) can be obtained by subjecting a dihydroxynaphthalene or a dihydroxyanthracene and a corresponding aldehyde or ketone to polycondensation reaction in the presence of an acid catalyst at normal pressure. In addition, a dissociation group or a crosslinking group can be introduced into at least one phenolic hydroxy group of that compound according to a publicly known method. If necessary, this reaction can also be carried out under increased pressure.

In (b) or (c) the method of subjecting a dihydroxynaphthalene or a dihydroxyanthracene and a corresponding aldehyde or ketone to polycondensation reaction in the presence of an acid catalyst in an aqueous solvent or with no solvent, the compound represented by the above formula (1) can be obtained by subjecting a dihydroxynaphthalene or a dihydroxyanthracene and a corresponding aldehyde or ketone to polycondensation reaction in the presence of an acid and a mercapto catalyst. In addition, a dissociation group can be introduced into at least one phenolic hydroxy group of that compound according to a publicly known method. Also, the present reaction can be carried out under reduced pressure, at normal pressure, or under increased pressure.

Various isomers can be used for the dihydroxynaphthalene, the dihydroxyanthracene, and the aldehyde or ketone.

Examples of the dihydroxynaphthalene include, but not particularly limited to, 1,2-dihydroxynaphthalene, 1,3-dihydroxynaphthalene, 1,4-dihydroxynaphthalene, 1,5-dihydroxynaphthalene, 1,6-dihydroxynaphthalene, 1,7-dihydroxynaphthalene, 1,8-dihydroxynaphthalene, 2,3-dihydroxynaphthalene, 2,6-dihydroxynaphthalene, and 2,7-dihydroxynaphthalene. These can be used alone as one kind or can be used in combination of two or more kinds. Among them, 2,6-dihydroxynaphthalene and 2,7-dihydroxynaphthalene are preferably used from the viewpoint of ease of production.

Examples of the dihydroxyanthracene include, but not particularly limited to, 1,5-dihydroxyanthracene, 1,6-dihydroxyanthracene, 1,7-dihydroxyanthracene, 1,8-dihydroxyanthracene, 2,3-dihydroxyanthracene, 2,6-dihydroxyanthracene, and 2,7-dihydroxyanthracene. These can be used alone as one kind or can be used in combination of two or more kinds. Among them, 2,6-dihydroxyanthracene and 2,7-dihydroxyanthracene are preferably used from the viewpoint of ease of production.

Examples of the aldehyde include, but not particularly limited to, 1-naphthaldehyde and 2-naphthaldehyde. These aldehydes can be used alone as one kind or can be used in combination of two or more kinds.

Examples of the ketone include, but not particularly limited to, 1-acetonaphthone, 2-acetonaphthone, 1-benzonaphthone, and 2-benzonaphthone. These aldehydes can be used alone as one kind or can be used in combination of two or more kinds.

In the present embodiment, it is preferable to use ketones from the viewpoint of achieving both high heat resistance and high transparency.

The acid catalyst that can be used in the reaction mentioned above can be arbitrarily selected for use from publicly known acid catalysts and is not particularly limited. Inorganic acids, organic acids, Lewis acids, and solid acids are widely known as such acid catalysts, and examples thereof include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferably used from the viewpoint of production such as easy availability and handleability. The acid catalysts can be used alone as one kind or can be used in combination of two or more kinds. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

The mercapto catalyst used in the reaction mentioned above can be arbitrarily selected for use from publicly known acid catalysts and is not particularly limited. As such a mercapto catalyst, an alkylthiol and a mercaptocarboxylic acid are widely known. Examples of the alkylthiol include an alkyl mercaptan having 1 to 12 carbon atoms, preferably n-octyl mercaptan, N-decyl mercaptan, and n-dodecyl mercaptan, and examples of the mercaptocarboxylic acid include, but not particularly limited to, 2-mercaptopropionic acid and 3-mercaptopropionic acid. Among them, from the viewpoint of production, N-octyl mercaptan, N-decyl mercaptan, and n-dodecyl mercaptan are preferable. The mercapto catalysts can be used alone as one kind or can be used in combination of two or more kinds. Also, the amount of the mercapto catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

The organic solvent or aqueous solvent used in (a) or (b) mentioned above, respectively, is not particularly limited as long as the reaction of the aldehyde or the ketone used with the dihydroxynaphthalene or the dihydroxyanthracene proceeds, and can be arbitrarily selected and used from publicly known solvents. Depending on the method, examples thereof include water, methanol, ethanol, propanol, butanol, tetrahydrofuran, dioxane, ethylene glycol dimethyl ether, ethylene glycol diethyl ether, and a mixed solvent thereof. The solvents can be used alone as one kind or can be used in combination of two or more kinds.

Also, the amount of these solvents used can be arbitrarily set according to, for example, the kind of the raw materials used and the catalyst used and moreover the reaction conditions and is not particularly limited, but is preferably in the range of 0 to 2000 parts by mass based on 100 parts by mass of the reaction raw materials. Furthermore, the reaction temperature in the above reaction can be arbitrarily selected according to the reactivity of the reaction raw materials and is not particularly limited, but is usually within the range of 10 to 200°C.

In order to obtain the compound represented by the formula (1) of the present embodiment, a higher reaction temperature is preferable. Specifically, the range of 60 to 200°C is preferable. The reaction method can be arbitrarily selected and used from publicly known approaches and is not particularly limited, and there are a method of charging the dihydroxynaphthalene or the dihydroxyanthracene, the aldehyde or the ketone, and the catalyst in one portion, and a method of dropping the dihydroxynaphthalene or the dihydroxyanthracene, and the aldehyde or the ketone, in the presence of the catalyst. After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, the compound that is the target compound can be obtained.

Examples of preferable reaction conditions mentioned above include using 1.0 mol to an excess of the dihydroxynaphthalene or the dihydroxyanthracene and 0.001 to 1 mol of the acid catalyst based on 1 mol of the aldehyde or the ketone, and reacting them at 50 to 150°C at normal pressure for about 20 minutes to 100 hours.

The target compound can be isolated by a publicly known method after the reaction terminates. The compound represented by the above formula (1) which is the target compound can be obtained, for example, by concentrating the reaction liquid, precipitating the reaction product by the addition of pure water, cooling the reaction liquid to room temperature, then separating the precipitates by filtration, filtering and drying the obtained solid matter, then separating and purifying the solid matter from by-products by column chromatography, and distilling off the solvent, followed by filtration and drying.

The compound represented by the formula (1) mentioned above can also be synthesized by (i') a method of subjecting a dihydroxynaphthalene or a dihydroxyanthracene and a corresponding aldehyde or ketone to polycondensation reaction in a base catalyst , instead of the method of subjecting to polycondensation reaction in the presence of an acid catalyst represented by (i) above.

The basic catalyst used in the method represented by (i') above can be arbitrarily selected for use from publicly known basic catalysts and is not particularly limited. As such a basic catalyst, inorganic bases and organic bases are known, and examples of inorganic bases can include an alkali metal hydroxide (sodium hydroxide, potassium hydroxide, etc.) and an alkali metal carbonate. Examples of the organic base include tertiary amines such as trialkylamines (trimethylamine, triethylamine, etc.), alkanolamines (triethanolamine, dimethylaminoethanol, etc.), alkoxides (sodium methoxide, sodium ethoxide, potassium t-butoxide, etc.), heterocyclic amines (morpholine, etc.), hexamethylenetetramine, diazabicycloundecene (DBU), diazabicyclononene (DBN), 1,4-diazabicyclo[2.2.2]octane (DABCO), and pyridine. Among them, sodium hydroxide, sodium methoxide, triethylamine, and DBU are preferable from the viewpoint of production such as easy availability and handleability. These basic catalysts may be used alone, or two or more thereof may be used in combination. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

Reaction conditions other than the basic catalyst used in the method represented by (i') above can be carried out in the same manner as the method of subjecting to polycondensation reaction in the presence of an acid catalyst represented by (i) above.

Alternatively, in the synthesis of the compound represented by the formula (1) mentioned above, (ii) a method of subjecting a methine site of a triarylmethane or a xanthene obtained by subjecting a dihydroxynaphthalene or a dihydroxyanthracene, respectively, and a corresponding aldehyde to polycondensation in the presence of an acid catalyst or a xanthene to substitution can be used instead of (i). In such a method, a compound (A) formed by replacing R^{Y} of the compound represented by the above formula (1) with a hydrogen atom is obtained by subjecting a dihydroxynaphthalene or a dihydroxyanthracene and a corresponding aldehyde or ketone to polycondensation reaction in the presence of an acid catalyst. Using a protective group introducing agent, a compound (B) is obtained in which a hydroxy group of the compound (A) is replaced with a protective group. Then, by allowing the hydrogen atom corresponding to the R^{Y} moiety of the compound represented by the above formula (1) to react with an alkylating agent in the presence of a basic catalyst, an alkyl group corresponding to the R^{Y} moiety of the compound represented by the above formula (1) is introduced. Further later, the protective group that has substituted the hydroxy group in the compound (B) is deprotected, thereby obtaining the above formula (1) in which R^{Y} is other than a hydrogen atom.

In the production method, for the method of introducing an alkyl group corresponding to the R^{Y} moiety of the compound represented by the above formula (1) into the hydrogen atom of the compound (B) corresponding to the R^{Y} moiety of the compound represented by the above formula (1), instead of allowing the hydrogen atom to react with an alkylating agent in the presence of a basic catalyst as in the above production method, the compound (B) may also be allowed to react with a halogenating agent to replace the hydrogen atom corresponding to the R^{Y} moiety of the compound represented by the above formula (1) with a halogen atom, and then allowed to react with an alkylating agent, thereby obtaining the above formula (1). The alkylating agent can be arbitrarily selected for use from publicly known alkylating agents and is not particularly limited. Examples thereof include a Grignard reagent, a halogenated hydrocarbon (methyl iodide, ethyl bromide, etc.), a dimethyl sulfate, an alkyllithium, and an alkylaluminum.

Alternatively, in the production method, for another method of introducing an alkyl group corresponding to the R^{Y} moiety of the compound represented by the above formula (1) into the hydrogen atom of the compound (B) corresponding to the R^{Y} moiety of the compound represented by the above formula (1), instead of allowing the hydrogen atom to react with an alkylating agent in the presence of a basic catalyst as in the above production method, the above formula (1) may also be obtained by replacing the hydrogen atom corresponding to the R^{Y} moiety of the compound represented by the above formula (1) with a hydroxyl group, and then allowed to react with an alkylating agent. Examples of the method of replacing the hydrogen atom corresponding to the R^{Y} moiety of the compound represented by the above formula (1) with a hydroxyl group include, but not particularly limited to, a method of oxidizing with lead dioxide and hydroxylating with sodium hydroxide in an acetic acid solvent. The alkylating agent can be arbitrarily selected for use from publicly known alkylating agents and is not particularly limited. Examples thereof include a Grignard reagent, a halogenated hydrocarbon (methyl iodide, ethyl bromide, etc.), a dimethyl sulfate, an alkyllithium, and an alkylaluminum.

In the polyphenol compound represented by the formula (1) or (2), a method for introducing a dissociation group or a crosslinking group into at least one phenolic hydroxyl group in the structure is publicly known. For example, "compound for introducing a dissociation group or a crosslinking group" can be used to introduce a dissociation group or a crosslinking group into at least one phenolic hydroxyl group. A compound for introducing the dissociation group or crosslinking group can be synthesized by a publicly known method or easily obtained. Examples thereof include, but not particularly limited to, acid chlorides, acid anhydrides, active carboxylic acid derivative compounds such as dicarbonates, alkyl halides, vinyl alkyl ethers, dihydropyran, and halocarboxylic acid alkyl esters.

In order to introduce a dissociation group or a crosslinking group into at least one phenolic hydroxyl group using "the compound for introducing a dissociation group or a crosslinking group", for example, the polyphenol compound is dissolved or suspended in an aprotic solvent such as acetone, tetrahydrofuran (THF), or propylene glycol monomethyl ether acetate. Subsequently, a vinyl alkyl ether such as ethyl vinyl ether, or dihydropyran is added to the solution or the suspension, and the mixture is reacted at 20 to 60°C at normal pressure for 6 to 72 hours in the presence of an acid catalyst such as pyridinium p-toluenesulfonate. The reaction liquid is neutralized with an alkali compound and added to distilled water to precipitate a white solid. Then, the separated white solid can be washed with distilled water and dried to obtain a compound in which a hydrogen atom of a hydroxy group is replaced with a dissociation group or a crosslinking group.

Alternatively, for example, the polyphenol compound having a hydroxy group is dissolved or suspended in an aprotic solvent such as acetone, THF, or propylene glycol monomethyl ether acetate. Subsequently, an alkyl halide such as ethyl chloromethyl ether or a halocarboxylic acid alkyl ester such as methyladamantyl bromoacetate is added to the solution or the suspension, and the mixture is reacted at 20 to 110°C at normal pressure for 6 to 72 hours in the presence of an alkali catalyst such as potassium carbonate. The reaction liquid is neutralized with an acid such as hydrochloric acid and added to distilled water to precipitate a white solid. Then, the separated white solid can be washed with distilled water and dried to obtain a polyphenol compound in which a hydrogen atom of a hydroxy group is replaced with a dissociation group or a crosslinking group.

As for the timing of introducing a dissociation group or a crosslinking group, the introduction may be carried out after condensation reaction of the dihydroxynaphthalene or dihydroxyanthracene with the aldehyde or the ketone or may be carried out at a stage previous to the condensation reaction. Alternatively, the introduction may be carried out after production of a resin, which will be mentioned later.

### (Physical properties and the like of composition for forming an optical component)

The composition of the present embodiment can form an amorphous film by a publicly known method such as spin coating.

### (Solvent)

Examples of the solvent to be used in the composition of the present embodiment can include, but not particularly limited to, an ethylene glycol monoalkyl ether acetate such as ethylene glycol monomethyl ether acetate, ethylene glycol monoethyl ether acetate, ethylene glycol mono-n-propyl ether acetate and ethylene glycol mono-n-butyl ether acetate; an ethylene glycol monoalkyl ether such as ethylene glycol monomethyl ether and ethylene glycol monoethyl ether; a propylene glycol monoalkyl ether acetate such as propylene glycol monomethyl ether acetate, propylene glycol monoethyl ether acetate (PGMEA), propylene glycol mono-n-propyl ether acetate and propylene glycol mono-n-butyl ether acetate; a propylene glycol monoalkyl ether such as propylene glycol monomethyl ether (PGME) and propylene glycol monoethyl ether; a lactate ester such as methyl lactate, ethyl lactate, n-propyl lactate, n-butyl lactate and n-amyl lactate; an aliphatic carboxylic acid ester such as methyl acetate, ethyl acetate, n-propyl acetate, n-butyl acetate, n-amyl acetate, n-hexyl acetate, methyl propionate and ethyl propionate; another ester such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutyl acetate, 3-methyl-3-methoxybutyl acetate, butyl 3-methoxy-3-methylpropionate, butyl 3-methoxy-3-methylbutyrate, methyl acetoacetate, methyl pyruvate and ethyl pyruvate; an aromatic hydrocarbon such as toluene and xylene; a ketone such as methyl ethyl ketone, 2-heptanone, 3-heptanone, 4-heptanone, cyclopentanone (CPN) and cyclohexanone (CHN); an amide such as N,N-dimethylformamide, N-methylacetamide, N,N-dimethylacetamide and N-methylpyrrolidone; and a lactone such as γ-lactone. These solvents can be used alone or in combination of two or more kinds.

The solvent used in the composition of the present embodiment is preferably a safe solvent, more preferably at least one selected from PGMEA, PGME, CHN, CPN, 2-heptanone, anisole, butyl acetate, ethyl propionate, and ethyl lactate, and still more preferably at least one selected from PGMEA, PGME, and CHN.

In the composition of the present embodiment, the relationship between the amount of the solid component and the amount of the solvent is not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the solid component and the solvent.

The composition of the present embodiment may contain at least one selected from the group consisting of an acid generating agent (C), a crosslinking agent (G), a basic compound (E), and a further component (F), as other solid components.

In the composition of the present embodiment, the content of the polyphenol compound (B) is not particularly limited, but is preferably 50 to 99.4% by mass of the entire mass of the solid components (summation of the polyphenol compound (B), and optionally used solid components such as acid generating agent (C), crosslinking agent (G), basic compound (E) and further component (F), hereinafter the same), more preferably 55 to 90% by mass, still more preferably 60 to 80% by mass, and particularly preferably 60 to 70% by mass.

### (Acid generating agent (C))

It is preferable that the composition of the present embodiment contain one or more acid generating agents (C) that directly or indirectly generate an acid by heat.

In the case of using the acid generating agent in the composition of the present embodiment, the content of the acid generating agent (C) is preferably 0.001 to 49% by mass of the entire mass of the solid components, more preferably 1 to 40% by mass, still more preferably 3 to 30% by mass, and particularly preferably 10 to 25% by mass. By using the acid generating agent (C) at a content within the range described above, even higher refractive index is obtained.

In the composition of the present embodiment, the acid generation method is not limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

Examples of the acid generating agent (C) include those described in International Publication No. WO 2013/024779. Among them, in particular, onium salts such as di-tertiary-butyl diphenyliodonium nonafluoromethanesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, tris(p-tert-butoxyphenyl) sulfonium trifluoromethanesulfonate, triphenylsulfonium p-toluenesulfonate, (p-tert-butoxyphenyl)diphenylsulfonium p-toluenesulfonate, tris(p-tert-butoxyphenyl)sulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl) sulfonium trifluoromethanesulfonate and 1,2'-naphthylcarbonylmethyltetrahydrothiophenium triflate; diazomethane derivatives such as bis(benzenesulfonyl)diazomethane, bis(p-toluenesulfonyl)diazomethane, bis(cyclohexylsulfonyl)diazomethane, bis(n-butylsulfonyl)diazomethane, bis(isobutylsulfonyl)diazomethane, bis(sec-butylsulfonyl)diazomethane, bis(n-propylsulfonyl)diazomethane, bis(isopropylsulfonyl)diazomethane and bis(tert-butylsulfonyl)diazomethane; glyoxime derivatives such as bis-(p-toluenesulfonyl)-α-dimethylglyoxime and bis-(n-butanesulfonyl)-α-dimethylglyoxime; bissulfone derivatives such as bisnaphthylsulfonylmethane; sulfonic acid ester derivatives of N-hydroxyimide compounds such as N-hydroxysuccinimide methanesulfonic acid ester, N-hydroxysuccinimide trifluoromethanesulfonic acid ester, N-hydroxysuccinimide 1-propanesulfonic acid ester, N-hydroxysuccinimide 2-propanesulfonic acid ester, N-hydroxysuccinimide 1-pentanesulfonic acid ester, N-hydroxysuccinimide p-toluenesulfonic acid ester, N-hydroxynaphthalimido methanesulfonic acid ester and N-hydroxynaphthalimido benzenesulfonic acid ester are preferably used.

### (Crosslinking agent (G))

It is preferable that the composition of the present embodiment contain one or more crosslinking agents (G) in the case of being used as an additive agent for increasing the strength of a structure. The crosslinking agent (G) is a compound capable of intramolecularly or intermolecularly crosslinking the polyphenol compound (B). Examples of such a crosslinking agent (G) can include, but not particularly limited to, a compound having one or more groups that are capable of crosslinking the polyphenol compound (B) (hereinafter, referred to as "crosslinkable groups").

The crosslinking agent is not particularly limited as long as it undergoes a crosslinking reaction, and any of publicly known crosslinking systems can be applied, but specific examples of the crosslinking agent that may be used in the present embodiment include, but are not particularly limited to, maleimide compounds, epoxy compounds, cyanate compounds, amino compounds, benzoxazine compounds, acrylate compounds, melamine compounds, guanamine compounds, glycoluril compounds, urea compounds, isocyanate compounds, azide compounds and the like. These crosslinking agents can be used alone as one kind or can be used in combination of two or more kinds.

In a crosslinking reaction between the polyphenol compound (B) and the crosslinking agent, for example, an active group of these crosslinking agents (a maleimide group, an epoxy group, a cyanate group, an amino group, or a phenolic hydroxy group formed by ring opening of the alicyclic site of benzoxazine) undergoes an addition reaction with the polyphenol compound (B) to form crosslinkage.

As the maleimide compound, a publicly known compound can be used. Examples thereof include aliphatic bismaleimide compounds such as N,N'-(2,2,4-trimethylhexamethylene)bismaleimide, N,N'-decamethylenebismaleimide, N,N'-octamethylenebismaleimide, N,N'-heptamethylenebismaleimide, N,N'-hexamethylenebismaleimide, N,N'-pentamethylenebismaleimide, N,N'-tetramethylenebismaleimide, N,N'-trimethylenebismaleimide, N,N'-ethylenebismaleimide, N,N'-(oxydimethylene)bismaleimide, 1,13-bismaleimide-4,7,10-trioxatridecane, and 1,11-bismaleimide-3,6,9-trioxaundecane; and aromatic maleimide compounds such as N,N'-(4-methyl-1,3-phenylene)bismaleimide, N,N'-(1,3-phenylene)bismaleimide, N,N'-(1,4-phenylene)bismaleimide, N,N'-(1,2-phenylene)bismaleimide, N,N'-(1,5-naphthylene)bismaleimide, N,N'-(4-chloro-1,3-phenylene)bismaleimide, N,N'-(methylene-p-phenylene)bismaleimide, N,N'-(4,4'-biphenylene)bismaleimide, N,N'-(sulfonyldi-p-phenylene)bismaleimide, N,N'-(oxydi-p-phenylene)bismaleimide, N,N'-(3,3'-dimethyl-4,4'-biphenylene)bismaleimide, N,N'-(benzylidenedi-p-phenylene)bismaleimide, N,N'-[methylenebis(3-chloro)-4-phenylene)]bismaleimide, N,N'-[methylenebis(3-methyl-4-phenylene)]bismaleimide, N,N'-[methylenebis(3-methoxy-4-phenylene)]bismaleimide, N,N'-(thiodi-p-phenylene)bismaleimide, N,N'-3,3'-benzophenonebismaleimide, N,N'-[methylenebis(3-methyl-5-ethyl-4-phenylene)]bismaleimide, N,N'-[tetramethylene bis(oxy-p-phenylene)]bismaleimide, 2,2-bis[4-(4-maleimide phenoxy)phenyl]propane, bis[4-(4-maleimide phenoxy)phenyl)] sulfone, 1,4-phenylene bis(4-maleimide phenoxy), bis[3-(4-maleimide phenoxy)phenyl] sulfone, bis[4-(3-maleimide phenoxy)phenyl]ketone, 1,3-phenylene bis(4-maleimide phenoxy), and bis[4-(4-maleimide phenylthio)phenyl]ether. Preferably, a bismaleimide is desirable from the viewpoint of heat resistance and solubility.

As the epoxy compound, a publicly known compound can be used and is selected from among compounds having two or more epoxy groups in one molecule. Examples thereof include those described in International Publication No. WO 2018/016614. These epoxy resins may be used alone or in combination of two or more kinds. An epoxy resin that is in a solid state at normal temperature, such as an epoxy resin obtained from a phenol aralkyl resin or a biphenyl aralkyl resin is preferable from the viewpoint of heat resistance and solubility.

The cyanate compound is not particularly limited as long as the compound has two or more cyanate groups in one molecule, and a publicly known compound can be used. Examples thereof include those described in WO 2011108524, but preferable examples of the cyanate compound in the present embodiment include cyanate compounds having a structure where hydroxy groups of a compound having two or more hydroxy groups in one molecule are replaced with cyanate groups. Also, the cyanate compound preferably has an aromatic group, and those having a structure in which a cyanate group is directly bonded to the aromatic group can be suitably used. Examples of such a cyanate compound include, but not particularly limited to, those described in International Publication No. WO 2018/016614. These cyanate compounds may be used alone or in arbitrary combination of two or more kinds. Also, the cyanate compound may be in any form of a monomer, an oligomer and a resin.

Examples of the amino compound include, but not particularly limited to, those described in International Publication No. WO 2018/016614.

The structure of oxazine of the benzoxazine compound is not particularly limited, and examples thereof include a structure of oxazine having an aromatic group including a condensed polycyclic aromatic group, such as benzoxazine and naphthoxazine.

Examples of the benzoxazine compound include compounds represented by the following general formulas (a) to (f). In the general formulas described below, a bond displayed toward the center of a ring indicates a bond to any carbon that constitutes the ring and to which a substituent can be bonded.

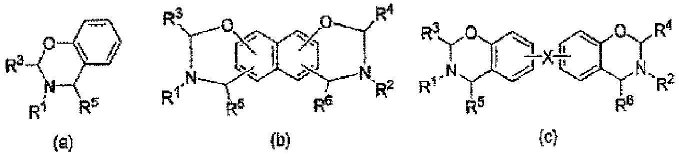

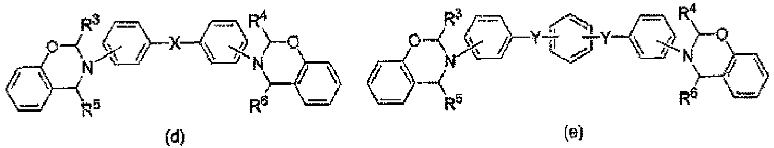

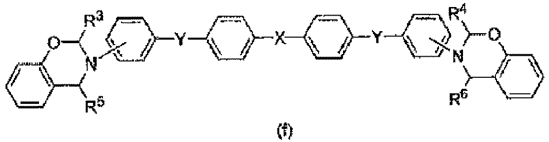

In the general formulas (a) to (c), R¹ and R² independently represent an organic group having 1 to 30 carbon atoms. In addition, in the general formulas (a) to (f), R³ to R⁶ independently represent hydrogen or a hydrocarbon group having 1 to 6 carbon atoms. Moreover, in the above general formulas (c), (d) and (f), X independently represents a single bond, -O-, -S-, -S-S-, -SO₂-, -CO-, -CONH-, -NHCO-, -C(CH₃)₂-, -C(CF₃)₂-,-(CH₂)m-, -O-(CH₂)m-O- or -S-(CH₂)m-S-. Here, m is an integer of 1 to 6. In addition, in the general formulas (e) and (f), Y independently represents a single bond,-O-, -S-, -CO-, -C(CH₃)₂-, -C(CF₃)₂- or alkylene having 1 to 3 carbon atoms.

Moreover, the benzoxazine compound includes an oligomer or polymer having an oxazine structure as a side chain, and an oligomer or polymer having a benzoxazine structure in the main chain.

The benzoxazine compound can be produced in a similar method as a method described in International Publication No. WO 2004/009708, Japanese Patent Application Laid-Open No. 11-12258 or Japanese Patent Application Laid-Open No. 2004-352670.

Specific examples of the melamine compound include those described in International Publication No. WO 2018/016614.

Specific examples of the guanamine compound include those described in International Publication No. WO 2018/016614.

Specific examples of the glycoluril compound include those described in International Publication No. WO 2018/016614.

Specific examples of the urea compound include those described in International Publication No. WO 2018/016614.

In the present embodiment, a crosslinking agent having at least one allyl group may be used from the viewpoint of improvement in crosslinkability. Specific examples of the crosslinking agent having at least one allyl group include, but particularly not limited to, those described in International Publication WO2018/016614. These crosslinking agents may be alone, or may be a mixture of two or more kinds. Among them, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide and bis(3-allyl-4-hydroxyphenyl) ether is preferable from the viewpoint of excellent compatibility with a biscitraconimide compound and/or an addition polymerization citraconimide resin.

With the composition for forming an optical component of the present embodiment, a film for forming an optical component of the present embodiment can be formed by crosslinking and curing the polyphenol compound (B) alone, or after compounding with the crosslinking agent by a publicly known method. Examples of the crosslinking method include approaches such as thermosetting and light curing.

In the composition of the present embodiment, the content of the crosslinking agent (G) is preferably 0.5 to 49% by mass of the entire mass of the solid components, more preferably 0.5 to 40% by mass, still more preferably 1 to 30% by mass, and particularly preferably 2 to 20% by mass. It is preferable to set the content ratio of the crosslinking agent (G) described above to 0.5% by mass or more because the inhibiting effect of the solubility of the composition for forming an optical component in an organic solvent can be improved. On the other hand, it is preferable to set the content ratio of the crosslinking agent (G) described above to 49% by mass or less because a decrease in heat resistance as the composition for forming an optical component can be suppressed.

### (Basic compound (E))

The composition of the present embodiment may contain a basic compound (E) having a function of controlling diffusion of an acid generated from an acid generating agent in the composition for forming an optical component to inhibit any unpreferable chemical reaction or the like. By using such a basic compound (E), the storage stability of the composition for forming an optical component is improved. Also, along with the further improvement of the resolution, the line width change of a structure due to variation in the post exposure delay time after heating can be inhibited, and the composition has extremely excellent process stability.

Such a basic compound (E) is not particularly limited, and examples thereof include a radiation degradable basic compound such as a nitrogen atom containing basic compound, a basic sulfonium compound and a basic iodonium compound. The basic compound (E) can be used alone or in combination of two or more kinds.

The basic compound (E) plays a role as a quencher against acids in order to prevent crosslinking reaction from proceeding due to a trace amount of an acid generated by the acid generating agent. Examples of such a basic compound include, but not particularly limited to, for example, primary, secondary or tertiary aliphatic amines, amine blends, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amide derivatives or imide derivatives, described in International Publication No. WO 2013-024779.

The content of the basic compound (E) is preferably 0.001 to 49% by mass of the entire mass of the solid components, more preferably 0.01 to 10% by mass, still more preferably 0.01 to 5% by mass, and particularly preferably 0.01 to 3% by mass. When the content of the basic compound (E) is within the above range, a decrease in resolution, and deterioration of the pattern shape and the dimension fidelity or the like can be further inhibited. Moreover, even though the post exposure delay time from electron beam irradiation to heating after radiation irradiation becomes longer, the shape of the pattern upper layer portion does not deteriorate. When the content of the basic compound (E) is 10% by mass or less, a decrease in sensitivity, and developability of the unexposed portion or the like can be prevented. Also, by using such a basic compound, the storage stability of the composition for forming an optical component is improved, the resolution is also improved, the line width change of the composition for forming an optical component due to variation in the post exposure delay time before radiation irradiation and the post exposure delay time after radiation irradiation can be inhibited, thereby making the composition extremely excellent in process stability.

### (Further component (F))

To the composition of the present embodiment, within the range of not inhibiting the purpose of the present embodiment, if required, as the further component (F), one kind or two kinds or more of various additive agents, such as a dissolution promoting agent, a dissolution controlling agent, a sensitizing agent, a surfactant and an organic carboxylic acid or an oxo acid of phosphorus or derivative thereof, can be added.

### -Dissolution promoting agent-

The low molecular weight dissolution promoting agent is a component having a function of, when the solubility of the polyphenol compound (B) in a developing solution is too low, increasing the solubility of the compound to moderately increase the dissolution rate of the compound upon developing. The low molecular weight dissolution promoting agent can be used to the extent that the effects of the present invention is not impaired. Examples of the dissolution promoting agent can include a low molecular weight phenolic compound, such as a bisphenol and tris(hydroxyphenyl)methane. These dissolution promoting agents can be used alone, or can be used as a mixture of two or more kinds. The content of the dissolution promoting agent, which is arbitrarily adjusted depending on the type of the compound containing the polyphenol compound (B) to be used, is preferably 0 to 49% by mass of the entire mass of the solid components, more preferably 0 to 5% by mass, still more preferably 0 to 1% by mass, and particularly preferably 0% by mass.

### -Dissolution controlling agent-

The dissolution controlling agent is a component having a function of, when the solubility of the polyphenol compound (B) in a developing solution is too high, controlling the solubility of the compound to moderately decrease the dissolution rate upon developing. As such a dissolution controlling agent, the one which does not chemically change in steps such as calcination of optical component, heating and development is preferable.

The dissolution controlling agent is not particularly limited, and examples thereof can include an aromatic hydrocarbon such as phenanthrene, anthracene and acenaphthene; a ketone such as acetophenone, benzophenone and phenyl naphthyl ketone; and a sulfone such as methyl phenyl sulfone, diphenyl sulfone and dinaphthyl sulfone. These dissolution controlling agents can be used alone, or can be used in combination of two or more kinds.

The content of the dissolution controlling agent, which is arbitrarily adjusted depending on the type of the polyphenol compound (B) to be used, is preferably, but not particularly limited to, 0 to 49% by mass of the entire mass of the solid components, more preferably 0 to 5% by mass, still more preferably 0 to 1% by mass, and particularly preferably 0% by mass.

### -Sensitizing agent-

The sensitizing agent is a component having a function of absorbing irradiated radiation energy, transmitting the energy to the acid generating agent (C), and thereby increasing the acid production amount, and improving the apparent sensitivity of a resist. Such a sensitizing agent is not particularly limited, and examples thereof can include a benzophenone, a biacetyl, a pyrene, a phenothiazine and a fluorene. These sensitizing agents can be used alone, or can be used in combination of two or more kinds. The content of the sensitizing agent, which is arbitrarily adjusted depending on the type of the polyphenol compound (B) to be used, is preferably 0 to 49% by mass of the entire mass of the solid components, more preferably 0 to 5% by mass, still more preferably 0 to 1% by mass, and particularly preferably 0% by mass.

### -Surfactant-

The surfactant is a component having a function of improving coatability and striation of the composition of the present embodiment, and the like. Such a surfactant is not particularly limited, and may be any of an anionic surfactant, a cationic surfactant, a nonionic surfactant and an amphoteric surfactant. A preferable surfactant is a nonionic surfactant. The nonionic surfactant has a good affinity with a solvent to be used in production of the composition for forming an optical component, and is more effective. Examples of the nonionic surfactant include, but not particularly limited to, a polyoxyethylene higher alkyl ether, a polyoxyethylene higher alkyl phenyl ether and a higher fatty acid diester of polyethylene glycol. Examples of commercially available products can include, hereinafter by trade name, EFTOP (manufactured by Jemco Inc.), MEGAFAC (manufactured by DIC Corporation), Fluorad (manufactured by Sumitomo 3M Limited), AsahiGuard, Surflon (hereinbefore, manufactured by Asahi Glass Co., Ltd.), Pepole (manufactured by Toho Chemical Industry Co., Ltd.), KP (manufactured by Shin-Etsu Chemical Co., Ltd.), and Polyflow (manufactured by Kyoeisha Chemical Co., Ltd.). The content of the surfactant, which is arbitrarily adjusted depending on the type of the resin containing the structural unit derived from the polyphenol compound (B) to be used, is preferably, but not particularly limited to, 0 to 49% by mass of the entire mass of the solid components, more preferably 0 to 5% by mass, still more preferably 0 to 1% by mass, and particularly preferably 0% by mass.

### -Organic carboxylic acid or oxo acid of phosphorus or derivative thereof-

For the purpose of prevention of sensitivity deterioration or improvement of a structure and post exposure delay stability or the like, and furthermore, as an optional component, the composition of the present embodiment may contain an organic carboxylic acid or an oxo acid of phosphorus or derivative thereof. These components can be used in combination with the basic compound, or may be used alone. The organic carboxylic acid is not particularly limited, and, for example, is suitably malonic acid, citric acid, malic acid, succinic acid, benzoic acid, salicylic acid, or the like. Examples of the oxo acid of phosphorus or derivative thereof include phosphoric acid or derivative thereof such as ester including phosphoric acid, di-n-butyl phosphate and diphenyl phosphate; phosphonic acid or derivative thereof such as ester including phosphonic acid, dimethyl phosphonate, di-n-butyl phosphonate, phenylphosphonic acid, diphenyl phosphonate and dibenzyl phosphonate; and phosphinic acid and derivative thereof such as ester including phosphinic acid and phenylphosphinic acid. Among the above, phosphonic acid is particularly preferable.

The organic carboxylic acid or the oxo acid of phosphorus or derivative thereof can be used alone, or can be used in combination of two or more kinds. The content of the organic carboxylic acid or the oxo acid of phosphorus or derivative thereof, which is arbitrarily adjusted depending on the type of the polyphenol compound (B) to be used, is preferably 0 to 49% by mass of the entire mass of the solid components, more preferably 0 to 5% by mass, still more preferably 0 to 1% by mass, and particularly preferably 0% by mass.

### -Further additive agent-

Furthermore, the composition of the present embodiment can contain one kind or two kinds or more of additive agents other than the dissolution controlling agent, sensitizing agent and surfactant described above, within the range of not inhibiting the purpose of the present invention, if required. Examples of such an additive agent include, but not particularly limited to, a dye, a pigment and an adhesion aid. For example, when the composition contains a dye or a pigment, a latent image of the exposed portion is visualized and influence of halation upon exposure can be alleviated, which is preferable. Also, when the optical component forming composition contains an adhesion aid, adhesiveness to a substrate can be improved, which is preferable. Furthermore, the further additive agent is not particularly limited, and examples thereof can include a halation preventing agent, a storage stabilizing agent, a defoaming agent and a shape improving agent. Specific examples thereof can include 4-hydroxy-4'-methylchalkone.

The total content of the optional component (F) is preferably 0 to 49% by mass of the entire mass of the solid components, more preferably 0 to 5% by mass, still more preferably 0 to 1% by mass, and particularly preferably 0% by mass.

In the composition of the present embodiment, the content of the polyphenol compound (B), the acid generating agent (C), the basic compound (E), the optional component (F) (the polyphenol compound (B)/the acid generating agent (C)/the basic compound (E)/the optional component (F)) is preferably 50 to 99.4/0.001 to 49/0.001 to 49/0 to 49 in % by mass based on the solid matter, more preferably 55 to 90/1 to 40/0.01 to 10/0 to 5, still more preferably 60 to 80/3 to 30/0.01 to 5/0 to 1, and particularly preferably 60 to 70/10 to 25/0.01 to 3/0.

The content ratio of each component is selected from each range so that the summation thereof is 100% by mass. When the content ratio is as described above, performance such as sensitivity, resolution and developability becomes further excellent.

The method for preparing the composition of the present embodiment is not particularly limited, and examples thereof include a method involving dissolving each component in a solvent upon use into a uniform solution, and then if required, filtering the solution through a filter or the like with a pore diameter of about 0.2 µm, for example.

The composition of the present embodiment may contain a resin within the range of not inhibiting the purpose of the present invention. Examples of the resin include, but not particularly limited to, a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of the resin is not particularly limited, and is arbitrarily adjusted according to the kind of the polyphenol compound (B) to be used, but is preferably 30 parts by mass or less per 100 parts by mass of the compound, more preferably 10 parts by mass or less, still more preferably 5 parts by mass or less, particularly preferably 0 parts by mass.

In addition, cured product obtained by curing the composition of the present embodiment can be used as a variety of resins. These cured products can be used for various applications as a highly versatile material imparting various characteristics such as high melting point, high refractive index and high transparency. The cured products mentioned above can be obtained by using subjecting the composition described above to a publicly known method corresponding to each composition, such as photoirradiation and heating.

These cured products can be used as a variety of synthetic resins such as an epoxy resin, a polycarbonate resin and an acrylic resin. Furthermore, they can be used as optical components such as lenses and optical sheets, recording materials such as hologram recording materials, organic photoreceptors (for example, a light emitting layer of a fluorescent element or the like), and highly functional materials such as photoresist materials, antireflection films, multilayer resist materials, and semiconductor sealing materials by taking advantage of their functionality.

### Examples

The present embodiment will be described in more detail with reference to synthesis examples, examples, and comparative examples below. However, the present embodiment is not limited to these examples by any means.

### (Synthesis Working Example 1) Synthesis of BiN-1

In a container (internal capacity: 300 mL) equipped with a stirrer, a condenser tube, and a burette, 6.2 g (38.8 mmol) of 2,7-dihydroxynaphthalene (a reagent manufactured by Sigma-Aldrich), 0.38 g of sulfuric acid, 3.0 g (19.2 mmol) of 2-naphthaldehyde (a reagent manufactured by Sigma-Aldrich), and 30.0 g of 1,4-dioxane were added, and the contents were reacted by being stirred at 100°C for 6 hours to obtain a reaction liquid. Next, 500 mL of pure water was added to the reaction liquid, followed by extraction with ethyl acetate. Next, the mixture was separated until neutral by the addition of pure water, and then concentrated to obtain a solution.

The obtained solution was separated by column chromatography to obtain 5.0 g of the objective compound (BiN-1) represented by the following formula (BiN-1).

As a result of measuring the molecular weight of the obtained compound (BiN-1) by the above method, it was 440. Also, the carbon concentration was 85% by mass, and the oxygen concentration was 11% by mass.

The following peaks were found by NMR measurement performed on the obtained compound (BiN-1) using 400 MHz-¹H-NMR with a solvent of DMSO-6, and the compound was confirmed to have a chemical structure of the following formula (BiN-1).
δ (ppm) 9.91 (2H, O-H), 6.97-8.17 (17H, Ph-H), 6.30 (1H, C-H)

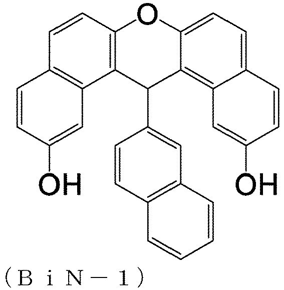

### (Synthesis Working Example 2) Synthesis of BiN-2

1-Naphthaldehyde was used instead of 2-naphthaldehyde, and the rest was carried out in the same manner as in Synthesis Working Example 1, whereby 5.5 g of the objective compound (BiN-2) represented by the following formula (BiN-2) was obtained.

As a result of measuring the molecular weight of the obtained compound (BiN-2) by the above method, it was 440. Also, the carbon concentration was 85% by mass, and the oxygen concentration was 11% by mass.

The following peaks were found by NMR measurement performed on the obtained compound (BiN-2) using 400 MHz-¹H-NMR with a solvent of DMSO-6, and the compound was confirmed to have a chemical structure of the following formula (BiN-2).
δ (ppm) 9.91 (2H, O-H), 6.97-8.17 (17H, Ph-H), 6.30 (1H, C-H)

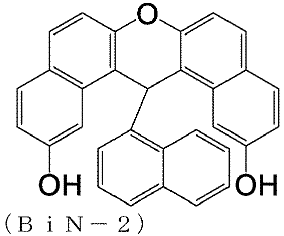

### (Synthesis Working Example 3) Synthesis of BiN-3

2,6-Dihydroxynaphthalene was used instead of 2,7-dihydroxynaphthalene, and the rest was carried out in the same manner as in Synthesis Working Example 1, whereby 4.0 g of the objective compound (BiN-3) represented by the following formula (BiN-3) was obtained.

As a result of measuring the molecular weight of the obtained compound (BiN-3) by the above method, it was 440. Also, the carbon concentration was 85% by mass, and the oxygen concentration was 11% by mass.

The following peaks were found by NMR measurement performed on the obtained compound (BiN-3) using 400 MHz-¹H-NMR with a solvent of DMSO-6, and the compound was confirmed to have a chemical structure of the following formula (BiN-3).
δ (ppm) 9.91 (2H, O-H), 6.97-8.17 (17H, Ph-H), 6.30 (1H, C-H)

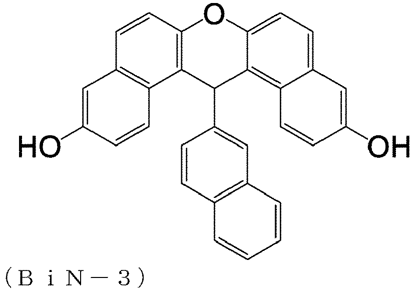

### (Synthesis Working Example 4) Synthesis of BiN-4

2-Acetonaphthone was used instead of 2-naphthaldehyde, and the rest was carried out in the same manner as in Synthesis Working Example 1, whereby 5.0 g of the objective compound (BiN-4) represented by the following formula (BiN-4) was obtained.

As a result of measuring the molecular weight of the obtained compound (BiN-4) by the above method, it was 440. Also, the carbon concentration was 85% by mass, and the oxygen concentration was 11% by mass.

The following peaks were found by NMR measurement performed on the obtained compound (BiN-4) using 400 MHz-¹H-NMR with a solvent of DMSO-6, and the compound was confirmed to have a chemical structure of the following formula (BiN-4).
δ (ppm) 9.56 (2H, O-H), 6.75-8.86 (17H, Ph-H), 2.75 (3H, C-H)

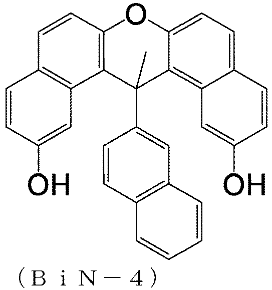

### (Synthesis Working Example 5) Synthesis of PBiN-1

In a container (internal capacity: 300 mL) equipped with a stirrer, a condenser tube, and a burette, 6.2 g (38.8 mmol) of 2,7-dihydroxynaphthalene (a reagent manufactured by Sigma-Aldrich), 0.38 g of sulfuric acid, 3.0 g (19.2 mmol) of 2-naphthaldehyde (a reagent manufactured by Sigma-Aldrich), and 30.0 g of 1,4-dioxane were added, and the contents were reacted by being stirred at 100°C for 6 hours to obtain a reaction liquid. Next, the reaction liquid was added to 1000 mL of a solvent having a ratio of methanol/hexane = 1/1, and the obtained solid was separated by filtration to obtain 8.0 g of the objective compound (PBiN-1) represented by the following formula (PBiN-1).

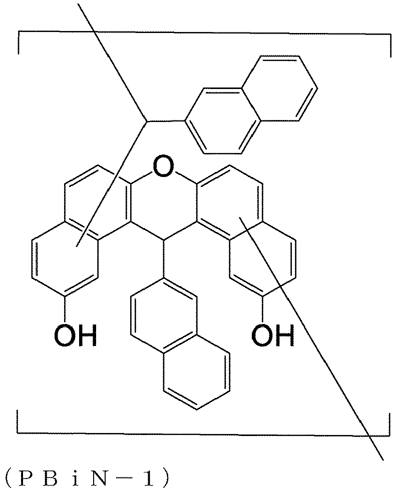

### (Synthesis Working Example 6) Synthesis of BiN-1U

In a container (internal capacity: 300 mL) equipped with a stirrer, a condenser tube, and a burette, 6.2 g (38.8 mmol) of 2,7-dihydroxynaphthalene (a reagent manufactured by Sigma-Aldrich), 0.38 g of sulfuric acid, 3.0 g (19.2 mmol) of 2-naphthaldehyde (a reagent manufactured by Sigma-Aldrich), and 30.0 g of 1,4-dioxane were added, and the contents were reacted by being stirred at 40°C for 2 hours to obtain a reaction liquid. Next, 500 mL of pure water was added to the reaction liquid, followed by extraction with ethyl acetate. Next, the mixture was separated until neutral by the addition of pure water, and then concentrated to obtain a solution.

The obtained solution was separated by column chromatography to obtain 5.5 g of the objective compound (BiN-1U) represented by the following formula (BiN-1U).

As a result of measuring the molecular weight of the obtained compound (BiN-1U) by the above method, it was 458.

The following peaks were found by NMR measurement performed on the obtained compound (BiN-1U) using 400 MHz-¹H-NMR with a solvent of DMSO-6, and the compound was confirmed to have a chemical structure of the following formula (BiN-1U).
δ (ppm) 9.2 (2H, O-H), 9.7 (2H, O-H), 6.8-7.9 (17H, Ph-H), 5.5 (1H, C-H)

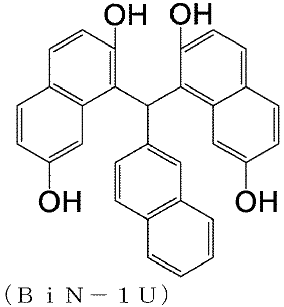

### (Synthesis Working Example 7) Synthesis of BiN-3U

2,6-Dihydroxynaphthalene was used instead of 2,7-dihydroxynaphthalene, and the rest was carried out in the same manner as in Synthesis Working Example 6, whereby 4.5 g of the objective compound (BiN-3U) represented by the following formula (BiN-3U) was obtained.

As a result of measuring the molecular weight of the obtained compound (BiN-3U) by the above method, it was 458.

The following peaks were found by NMR measurement performed on the obtained compound (BiN-3U) using 400 MHz-¹H-NMR with a solvent of DMSO-6, and the compound was confirmed to have a chemical structure of the following formula (BiN-3U).
δ (ppm) 9.2 (2H, O-H), 9.6 (2H, O-H), 6.7-8.0 (17H, Ph-H), 5.5 (1H, C-H)

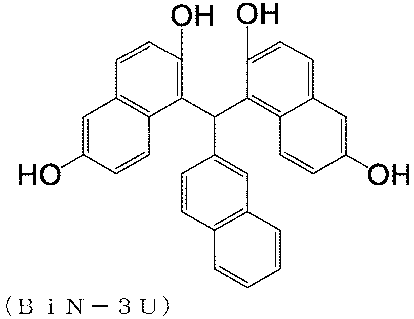

### (Synthesis Working Example 8) Synthesis of BiN-5U

2,3-Dihydroxynaphthalene was used instead of 2,7-dihydroxynaphthalene, and the rest was carried out in the same manner as in Synthesis Working Example 6, whereby 1.0 g of the objective compound (BiN-5U) represented by the following formula (BiN-5U) was obtained.

As a result of measuring the molecular weight of the obtained compound (BiN-5U) by the above method, it was 458.

The following peaks were found by NMR measurement performed on the obtained compound (BiN-5U) using 400 MHz-¹H-NMR with a solvent of DMSO-6, and the compound was confirmed to have a chemical structure of the following formula (BiN-5U).
δ (ppm) 9.4-9.6 (4H, O-H), 7.0-8.0 (17H, Ph-H), 5.5 (1H, C-H)

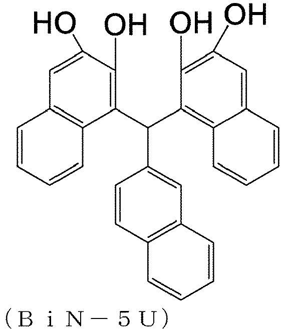

### (Synthesis Working Example 9) Synthesis of PBiN-3

In a container (internal capacity: 300 mL) equipped with a stirrer, a condenser tube, and a burette, 6.2 g (38.8 mmol) of 2,6-dihydroxynaphthalene (a reagent manufactured by Sigma-Aldrich), 1.12 g of methanesulfonic acid, 3.0 g (19.2 mmol) of 2-naphthaldehyde (a reagent manufactured by Sigma-Aldrich), and 30.0 g of 1,4-dioxane were added, and the contents were reacted by being stirred at 100°C for 6 hours to obtain a reaction liquid.

The obtained solution was added to 1000 mL of a solvent having a ratio of methanol/hexane = 1/1, and the obtained solid was separated by filtration to obtain 5.5 g of the objective compound (PBiN-3) represented by the following formula (PBiN-3).

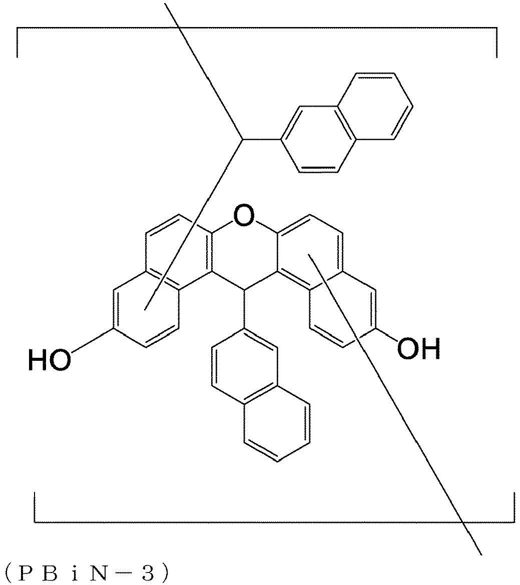

### (Synthesis Working Example 10) Synthesis of BiN-3-MeBOC

To a container (internal capacity: 200 mL) equipped with a stirrer, a condenser tube, and a burette, 5.2 g (11.8 mmol) of the compound (BiN-3) obtained as described above, 5.4 g (27 mmol) of t-butyl bromoacetate (manufactured by Sigma-Aldrich), and 100 mL of acetone were charged, and 3.8 g (27 mmol) of potassium carbonate (manufactured by Sigma-Aldrich) and 0.8 g of 18-crown-6 were added. The contents were stirred for 3 hours under reflux and reacted. Next, after the reaction terminates, the reaction liquid was concentrated, and the reaction product was precipitated by adding 100 g of pure water to the concentrate, cooled to room temperature, and then filtered to separate solid matter.

The obtained solid matter was dried, and then separated and purified by column chromatography to obtain 1.0 g of the following formula (BiN-3-MeBOC).

The following peaks were found by NMR measurement performed on the obtained compound (BiN-3-MeBOC) under the above measurement conditions, and the compound was confirmed to have a chemical structure of the following formula (BiN-3-MeBOC).
1H-NMR (d6-DMSO): δ (ppm) 1.4 (18H, O-C-CH3), 4.9 (4H, O-CH2-C), 6.9-8.2 (17H, Ph-H), 6.3 (1H, C-H).

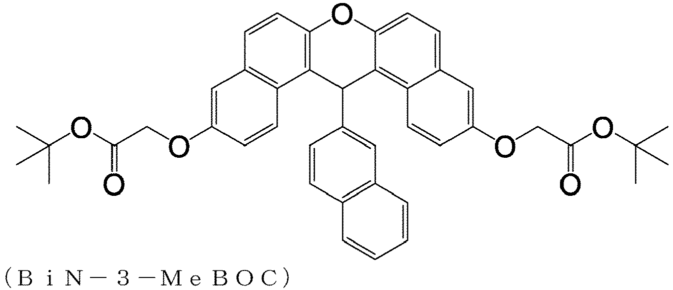

### (Synthesis Working Example 11) Synthesis of BiN-3-AL

To a container (internal capacity: 1000 mL) equipped with a stirrer, a condenser tube, and a burette, 11 g (25 mmol) of the compound (BiN-3) obtained as described above, 108 g (810 mmol) of potassium carbonate, and 200 mL of dimethylformamide were charged, and 185 g (1.53 mol) of allyl bromide was added thereto, and the reaction liquid was reacted by being stirred at 110°C for 24 hours. Next, the reaction liquid was concentrated. The reaction product was precipitated by the addition of 500 g of pure water. After cooling to room temperature, the precipitates were separated by filtration. The obtained solid matter was filtered and dried and then separated and purified by column chromatography to obtain 7.2 g of the objective compound (BiN-3-AL) represented by the following formula.

The following peaks were found by NMR measurement performed on the obtained compound under the above measurement conditions, and the compound was confirmed to have a chemical structure of the following formula (BiN-3-AL) .
1H-NMR: (d6-DMSO, internal standard TMS): δ (ppm) 6.9-8.2 (17H, Ph-H), 6.3 (1H, C-H), 6.0 (2H, -CH=CH2), 5.2-5.4 (4H, -CH=CH2), 4.4 (4H, -CH2-)

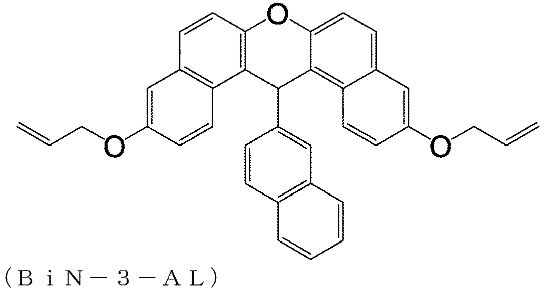

### (Synthesis Working Example 12) Alternative Synthesis of BiN-4

In a container (internal capacity: 2 L) equipped with a stirrer, a condenser tube, and a buret, 66.7 g (151 mmol) of BiN-1 obtained in Synthesis Working Example 1 was dissolved in 450 mL of DMF, and 63 g (456 mmol) of potassium carbonate and 38 mL (610 mmol) of methyl iodide were added sequentially under ice cooling. After the reaction at room temperature for 12 hours, the reaction liquid was added to pure water to obtain a solid matter. The solid matter was washed and dried to obtain 79.5 g of an intermediate 1 represented by the following formula (intermediate 1).

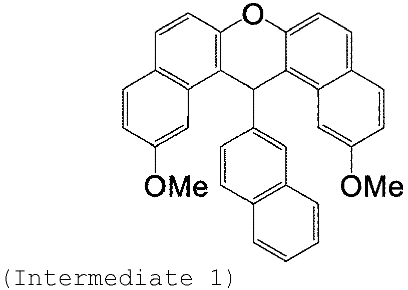

Next, in a container (internal capacity: 2 L) equipped with a stirrer, a condenser tube, and a burette, 59.2 g (126 mmol) of the compound represented by the formula (intermediate 1) was dissolved in 1200 mL of acetic acid, and 45 g (190 mmol) of lead dioxide was added. The mixture was stirred at 120°C for 2 hours, allowed to cool to room temperature, and the reaction liquid was added to a 25% aqueous sodium hydroxide solution to obtain a solid matter. The obtained solid matter was washed and dried to obtain 52.9 g of an intermediate 2 represented by the following formula intermediate 2).

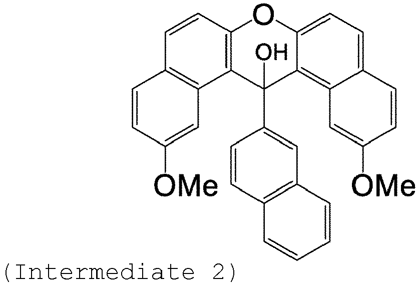

Next, in a container (internal capacity: 2 L) equipped with a stirrer, a condenser tube, and a burette, 62.5 g (129 mmol) of the compound represented by the formula (intermediate 2) was dissolved in 650 mL of toluene and added thereto, and 140 mL of a 1.4 M-trimethylaluminum-hexane solution (manufactured by Tokyo Kasei Kogyo Co., Ltd.) was added dropwise at room temperature, and the mixture was stirred at 100°C for 2 hours. After the reaction terminates, the reaction liquid was cooled under ice cooling and added to water. Then, the organic layer obtained by filtration and separation was washed and dried, and the solvent was distilled off to obtain a solid matter. The obtained solid matter was washed and dried to obtain 52.9 g of an intermediate 2 represented by the following formula intermediate 2). The obtained solid was separated by column chromatography to obtain 18.3 g of an intermediate 3 represented by the following formula (intermediate 3).

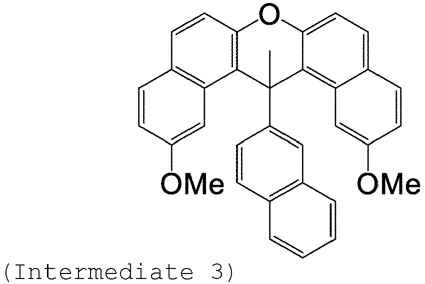

Further, in a container (internal capacity: 1 L) equipped with a stirrer, a condenser tube, and a burette, 18.3 g (38.0 mmol) of the compound represented by the formula (intermediate 3) was dissolved in 200 mL of dichloromethane and added thereto, and 120 mL of a 1.0 M-tribromoborone-dichloromethane solution (manufactured by Tokyo Kasei Kogyo Co., Ltd.) was added dropwise under ice cooling, and after dropping, the mixture was stirred at room temperature for 12 hours, and water was added to stop the reaction. After the reaction terminates, the filtrate obtained by filtering the reaction liquid was dissolved in ethyl acetate, and the solvent was distilled off after washing and dehydration to obtain 12.5 g of the target compound BiN-4 represented by the following formula (BiN-4). The obtained BiN-4 was confirmed to have a structure of the formula (BiN-4) in the same manner as in Synthesis Working Example 4.

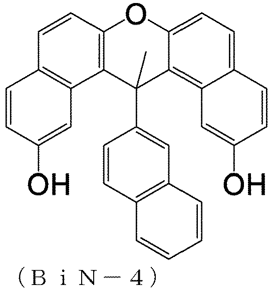

### (Synthesis Working Example 13) Synthesis of BiN-1-Ac

In the same manner as in Synthesis Working Example 11, 7.0 g of the objective compound (BiN-1-Ac) represented by the following formula was obtained, except that the compound (BiN-1) was used instead of the compound (BiN-3) mentioned above, and 110 g (1.53 mol) of acrylic acid was used instead of 186 g (1.53 mol) of allyl bromide mentioned above.

The following peaks were found by NMR measurement performed on the obtained compound under the above measurement conditions, and the compound was confirmed to have a chemical structure of the following formula (BiN-1-Ac) .
1H-NMR: (d6-DMSO, internal standard TMS): δ (ppm) 6.8-8.0 (17H, Ph-H), 6.2 (2H, =C-H), 6.1 (2H, -CH=C), 5.7 (2H, =C-H), 5.3 (1H, C-H)

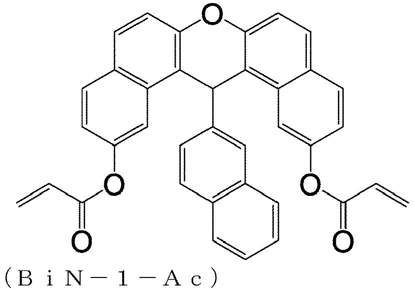

### (Synthesis Working Example 14) Synthesis of BiN-1-Ea

To a container (internal capacity: 100 ml) equipped with a stirrer, a condenser tube, and a burette, 9.2 g (20 mmol) of the compound represented by the formula (BiN-1) mentioned above, 6.1 g of glycidyl methacrylate, 0.5 g of triethylamine, and 0.05 g of p-methoxyphenol were charged to 50 ml of methyl isobutyl ketone, and the contents were warmed to 80°C and reacted by being stirred for 24 hours.

The resultant was cooled to 50°C, and the reaction liquid was added dropwise into pure water. The precipitated solid matter was filtered, dried, and then separated and purified by column chromatography to obtain 3.0 g of the objective compound represented by the following formula (BiN-1-Ea).

The obtained compound was confirmed to have a chemical structure of the following formula (BiN-1-Ea) by 400 MHz-¹H-NMR.
1H-NMR: (d-DMSO, internal standard TMS): δ (ppm) 6.8-8.0 (17H, Ph-H), 6.4-6.5 (4H, C=CH2), 5.3 (1H, C-H), 5.7 (2H, -OH), 4.7 (2H, C-H), 4.0-4.4 (8H, -CH2-), 2.0 (6H, -CH3)

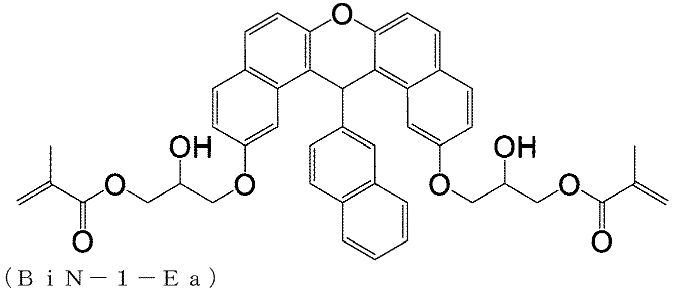

### (Synthesis Working Example 15) Synthesis of BiN-1-Ua

To a container (internal capacity: 100 mL) equipped with a stirrer, a condenser tube, and a burette, 9.2 g (20 mmol) of the compound represented by the formula (BiN-1) mentioned above, 6.1 g of 2-isocyanatoethyl methacrylate, 0.5 g of triethylamine, and 0.05 g of p-methoxyphenol were added to 50 mL of methyl isobutyl ketone, and the contents were warmed to 80°C and reacted by being stirred for 24 hours. The resultant was cooled to 50°C, and the reaction liquid was added dropwise into pure water. The precipitated solid matter was filtered, dried, and then separated and purified by column chromatography to obtain 3.0 g of the objective compound represented by the following formula (BiN-1-Ua). The obtained compound was confirmed to have a chemical structure of the following formula (BiN-1-Ua) by 400 MHz-¹H-NMR.
1H-NMR: (d-DMSO, internal standard TMS): δ (ppm) 8.8 (4H, -NH2), 6.8-8.0 (17H, Ph-H), 6.4-6.5 (4H, =CH2), 5.3 (1H, C-H), 4.1 (4H, -CH2-), 3.4 (2H, C-H) 2.2 (4H, -CH2-), 2.0 (6H, -CH3)

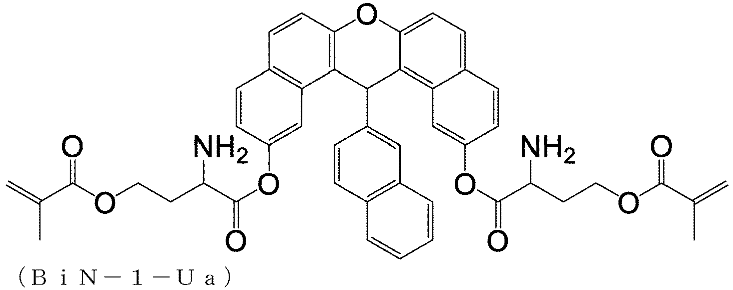

### (Synthesis Working Example 16) Synthesis of BiN-1-E

To a container (internal capacity: 100 mL) equipped with a stirrer, a condenser tube, and a burette, 9.2 g (20 mmol) of the compound represented by the formula (BiN-1) mentioned above and 14.8 g (107 mmol) of potassium carbonate were charged with 50 mL of dimethylformamide, and 6.56 g (54 mmol) of acetic acid-2-chloroethyl was added thereto, and the reaction liquid was reacted by being stirred at 90°C for 12 hours. Next, the reaction liquid was cooled in an ice bath to precipitate crystals, which were then separated by filtration. Subsequently, to a container (internal capacity: 100 mL) equipped with a stirrer, a condenser tube, and a burette, 40 g of the crystals mentioned above, 40 g of methanol, 100 g of THF and a 24% aqueous sodium hydroxide solution were charged, and the reaction liquid was reacted by being stirred for 4 hours under reflux. Then, the reaction liquid was cooled in an ice bath and concentrated. The precipitated solid matter was filtered, dried, and then separated and purified by column chromatography to obtain 5.2 g of the objective compound represented by the following formula (BiN-1-E). The obtained compound was confirmed to have a chemical structure of the following formula (BiN-1-E) by 400 MHz-¹H-NMR.
1H-NMR: (d-DMSO, internal standard TMS): δ (ppm) 6.8-7.9 (17H, Ph-H), 5.3 (1H, C-H), 4.9 (2H, -OH), 4.4 (4H,-CH2-), 3.7 (4H, -CH2-)

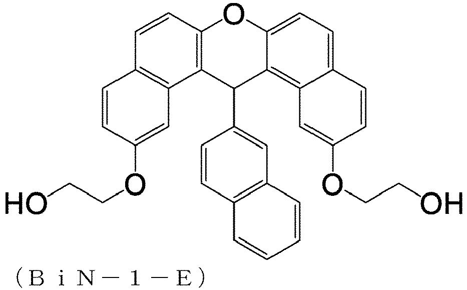

### (Synthesis Working Example 17) Synthesis of BiN-1-PX

To a container (internal capacity: 1000 mL) equipped with a stirrer, a condenser tube, and a burette, 37 g (84 mmol) of the compound represented by the formula (BiN-1) mentioned above, 62.9 g of iodoanisole, 116.75 g of cesium carbonate, 1.88 g of dimethylglycine hydrochloride, and 0.68 g of copper iodide were charged with 400 mL of 1,4-dioxane, and the contents were warmed to 95°C and reacted by being stirred for 22 hours. Next, insoluble matter was filtered off, and the filtrate was concentrated and added dropwise into pure water. The precipitated solid matter was filtered, dried, and then separated and purified by column chromatography to obtain 1.6 g of an intermediate compound represented by the following formula (BiN-1-M).

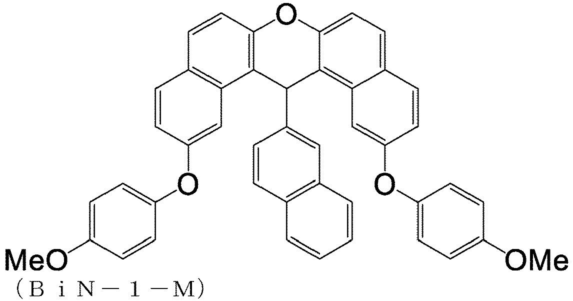

Next, to a container (internal capacity: 1000 mL) equipped with a stirrer, a condenser tube, and a burette, 16.0 g of the compound represented by the formula (BiN-1-M) mentioned above and 80 g of pyridine hydrochloride were added, and the contents were reacted by being stirred at 190°C for 2 hours. Next, 160 mL of hot water was further added thereto, and the mixture was stirred to precipitate solid matter. Then, 250 mL of ethyl acetate and 100 mL of water were added thereto, and the mixture was stirred, left to stand still, and separated. The organic layer was concentrated, dried, and then separated and purified by column chromatography to obtain 11.5 g of the objective compound represented by the following formula (BiN-1-PX).

The obtained compound was confirmed to have a chemical structure of the following formula (BiN-1-PX) by 400 MHz-¹H-NMR.
1H-NMR: (d-DMSO, internal standard TMS): δ (ppm) 9.1 (2H, O-H), 6.8-8.0 (25H, Ph-H), 5.3 (1H, C-H)

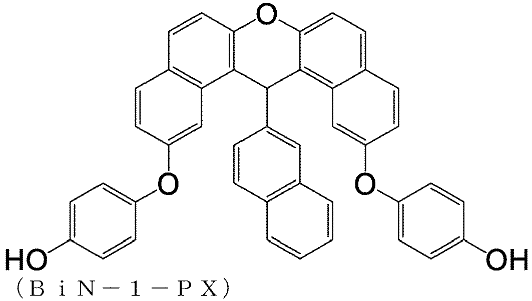

### (Synthesis Working Example 18) Synthesis of BiN-1-PE

The same reaction as in Synthesis Working Example 1-7 was performed except that the compound represented by the formula (BiN-1-E) mentioned above was used instead of the compound represented by the formula (BiN-1) mentioned above to obtain 4.2 g of the objective compound represented by the following formula (BiN-1-PE).

The obtained compound was confirmed to have a chemical structure of the following formula (BiN-1-PE) by 400 MHz-¹H-NMR.
1H-NMR: (d-DMSO, internal standard TMS): δ (ppm) 9.1 (2H, O-H), 6.7-8.0 (25H, Ph-H), 5.3 (1H, C-H), 4.4 (4H, - CH2-), 3.1 (4H, -CH2-)

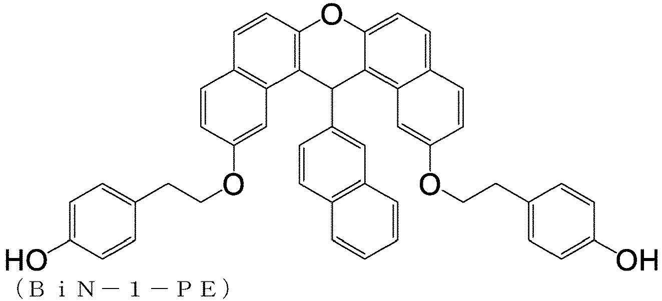

### (Synthesis Working Example 19) Synthesis of BiN-1-G

To a container (internal capacity: 100 mL) equipped with a stirrer, a condenser tube, and a burette, a liquid prepared by adding 9.2 g (21 mmol) of the compound represented by the above formula (BiN-1) and 6.2 g (45 mmol) of potassium carbonate to 100 mL of dimethylformamide were charged, and then 4.1 g (45 mmol) of epichlorohydrin was added thereto, and the obtained reaction liquid was reacted by being stirred at 90°C for 6.5 hours. Next, the solid content was then removed from the reaction liquid by filtration, cooled in an ice bath, crystals were precipitated, filtered, dried, and then separated and purified by column chromatography to obtain 3.8 g of the objective compound represented by the following formula (BiN-1-G).

The following peaks were found by NMR measurement performed on the obtained compound (BiN-1-G) under the above measurement conditions mentioned above, and the compound was confirmed to have a chemical structure of the following formula (BiN-1-G).
1H-NMR: (d-DMSO, internal standard TMS): δ (ppm) 6.8-8.0 (17H, Ph-H), 5.3 (C-H), 4.0-4.3 (4H, -CH2-), 2.3-3.0 (6H, -CH (CH2) O)

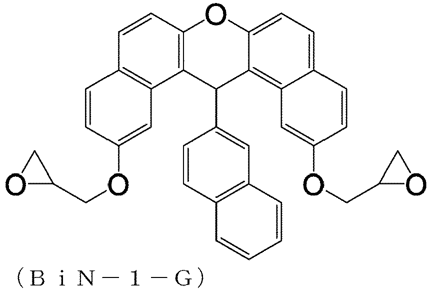

### (Synthesis Working Example 20) Synthesis of BiN-1-GE

The same reaction as in Synthesis Working Example 19 was performed except that the compound represented by the above formula (BiN-1-E) was used instead of the compound represented by the above formula (BiN-1-E) to obtain 3.0 g of the objective compound represented by the following formula (BiN-1-GE).

The compound was confirmed to have a chemical structure of the following formula (BiN-1-GE) by 400 MHz-¹H-NMR .
1H-NMR: (d-DMSO, internal standard TMS): δ (ppm) 6.8-8.0 (17H, Ph-H), 5.3 (C-H), 3.3-4.4 (12H, -CH2-), 2.3-2.8 (6H, -CH (CH2) O)

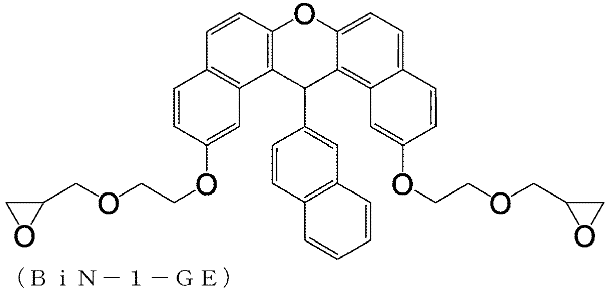

### (Synthesis Working Example 21) Synthesis of BiN-1-SX

To a container (internal capacity: 100 mL) equipped with a stirrer, a condenser tube, and a burette, 9.2 g (21 mmol) of the compound represented by the above formula (BiN-1) and 6.4 g of vinyl benzyl chloride (trade name CMS-P; manufactured by Seimi Chemical Co., Ltd.) were charged with 50 mL of dimethylformamide, and the contents were warmed to 50°C and stirred, and 8.0 g of 28% by mass of sodium methoxide (methanol solution) was added thereto from a dropwise funnel over 20 minutes, and the reaction liquid was reacted by being stirred at 50°C for 1 hour. Next, 1.6 g of 28% by mass of sodium methoxide (methanol solution) was added, and the reaction solution was warmed to 60°C and stirred for 3 hours, and 1.2 g of 85% by mass of phosphoric acid was further added, stirred for 10 minutes, and then the resultant was cooled to 40°C, and the reaction liquid was added dropwise into pure water. The precipitated solid matter was filtered, dried, and then separated and purified by column chromatography to obtain 3.5 g of the objective compound represented by the following formula (BiN-1-SX).

The obtained compound was confirmed to have a chemical structure of the following formula (BiN-1-SX) by 400 mhz-¹H-NMR.
1H-NMR: (d-DMSO, internal standard TMS): Δ (ppm) 6.8-7.9 (25H, Ph-H), 5.2-5.8 (11H, -CH2-, -CH=CH2, C-H)

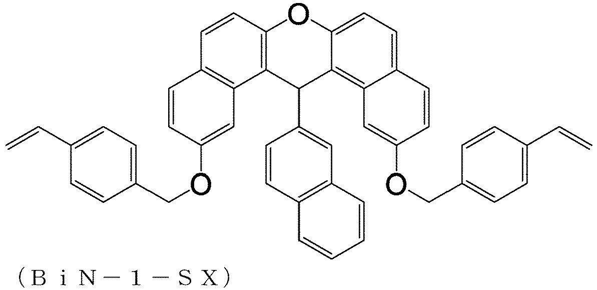

### (Synthesis Working Example 22) Synthesis of BiN-1-SE

The same reaction as in Synthesis Working Example 20 was performed except that the compound represented by the above formula (BiN-1-E) was used instead of the compound represented by the above formula (BiN-1) to obtain 3.5 g of the objective compound represented by the following formula (BiN-1-SE).

The obtained compound was confirmed to have a chemical structure of the following formula (BiN-1-SE) by 400 mhz-¹H-NMR.
1H-NMR: (d-DMSO, internal standard TMS): Δ (ppm) 7.0-8.0 (25H, Ph-H), 3.8-6.7 (19H, -CH2-CH2-, -CH2-, -CH=CH2, C-H)

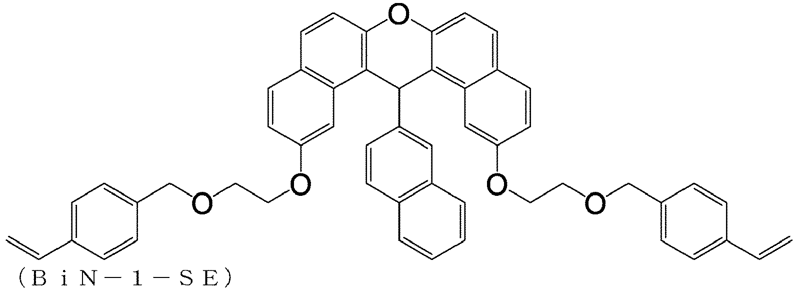

### (Synthesis Working Example 23) Synthesis of BiN-1-Pr

In a container (internal capacity: 300 mL) equipped with a stirrer, a condenser tube, and a burette, 8.8 g (20 mmol) of a compound represented by the above formula (BiN-1) and 7.9 g (66 mmol) of propargyl bromide were charged to 100 mL of dimethylformamide, and the contents were reacted by being stirred at room temperature for 3 hours to obtain a reaction liquid. Next, the reaction liquid was concentrated, and the reaction product was precipitated by the addition of 300 g of pure water to the concentrate, cooled to room temperature, and then filtered to separate solid matter.

The obtained solid matter was filtered, dried, and then separated and purified by column chromatography, thereby obtaining 6.0 g of the objective compound (BiN-1-Pr) represented by the following formula (BiN-1-Pr).

The following peaks were found by NMR measurement performed on the obtained compound (BiN-1-Pr) under the above measurement conditions mentioned above, and the compound was confirmed to have a chemical structure of the following formula (BiN-1-Pr).
δ (ppm): 6.8-7.9 (17H, Ph-H), 5.3 (1H, C-H), 4.8 (4H, - CH2-), 2.1 (2H, ≡CH)

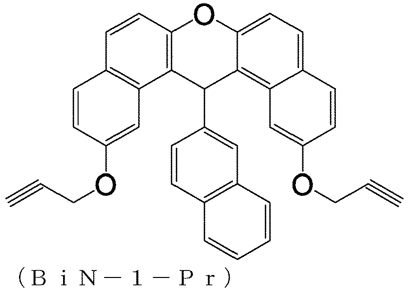

### (Synthesis Example 1)

A four necked flask (internal capacity: 10 L) equipped with a Dimroth condenser tube, a thermometer and a stirring blade, and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were charged in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction liquid, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

The molecular weight of the obtained dimethylnaphthalene formaldehyde was as follows: Mn: 562, Mw: 1168, and Mw/Mn: 2.08.

Subsequently, a four necked flask (internal capacity: 0.5 L) equipped with a Dimroth condenser tube, a thermometer and a stirring blade was prepared. To this four necked flask, 100 g (0.51 mol) of the dimethylnaphthalene formaldehyde resin obtained as mentioned above, and 0.05 g of p-toluenesulfonic acid were charged in a nitrogen stream, and the temperature was raised to 190°C at which the mixture was then heated for 2 hours, followed by stirring. Subsequently, 52.0 g (0.36 mol) of 1-naphthol was further added thereto, and the temperature was further raised to 220°C at which the mixture was reacted for 2 hours. After dilution with a solvent, neutralization and washing with water were performed, and the solvent was distilled off under reduced pressure to obtain 126.1 g of a modified resin (CR-1) as a black-brown solid.

The obtained resin (CR-1) had Mn: 885, Mw: 2220, and Mw/Mn: 2.51. Also, the carbon concentration was 89.1% by mass, and the oxygen concentration was 4.5% by mass.

### (Examples 1 to 23 and Comparative Example 1)

With respect to the solubility of each compound in an organic solvent, the solubility of the compound in propylene glycol monomethyl ether was measured. The solubility was evaluated according to the following criteria, using the amount of dissolution in propylene glycol monomethyl ether. For the measurement of the amount of dissolution, the evaluation was carried out by precisely weighing the compound in a test tube at 23°C, adding propylene glycol monomethyl ether acetate to a predetermined concentration, applying ultrasonic waves for 30 minutes with an ultrasonic cleaning machine, visually observing the subsequent state of the liquid, and determining the concentration of the completely dissolved amount as the basis.

The compositions for forming optical components were each prepared so that the amount of the compound shown in the following table was 5% by mass in propylene glycol methyl ether. Next, a silicon substrate was spin coated with each of these compositions for forming an optical component, and then baked at 110°C for 60 seconds to prepare an optical component forming film each having a film thickness of 1000 nm.

Then, tests for the refractive index and the transparency at a wavelength of 633 nm were carried out by using a vacuum ultraviolet with variable angle spectroscopic ellipsometer (model name: VUV-VASE) manufactured by J.A. Woollam Japan, and the refractive index and the transparency were evaluated. The optical component forming film was baked at 110°C for 5 minutes to evaluate the film heat resistance.

The evaluation results are shown in the following table.

### [Solubility test]

A: 5.0% by mass ≤ amount of dissolution
B: 3.0% by mass ≤ amount of dissolution < 5.0% by mass
C: amount of dissolution < 3.0% by mass

### [Evaluation criteria for refractive index]

A: The refractive index is 1.75 or more.
B: The refractive index is 1.65 or more and less than 1.75.
C: The refractive index is less than 1.65.

### [Evaluation criteria for transparency]

A: The absorption coefficient is less than 0.03.
B: The absorption coefficient is 0.03 or more and less than 0.05.
C: The absorption coefficient is 0.05 or more.

### [Evaluation criteria for film heat resistance]

A: No visual defects in the film
C: Visual defects in the film (including the loss of films)

### [Table 1]

**Table 1**

| | Compound | Solubility test | Evaluation of refractive index | Evaluation of transparency | Evaluation of film heat resistance |
|---|---|---|---|---|---|
| Example 1 | BiN-1 | A | A | A | A |
| Example 2 | BiN-2 | A | A | A | A |
| Example 3 | BiN-3 | A | A | A | A |
| Example 4 | BiN-4 | A | A | A | A |
| Example 5 | PBiN-1 | A | A | A | A |
| Example 6 | BiN-1U | A | A | A | A |
| Example 7 | BiN-3U | A | A | A | A |
| Example 8 | BiN-5U | A | A | A | A |
| Example 9 | PBiN-3 | A | A | A | A |
| Example 10 | BiN-3-MeBOC | A | B | A | A |
| Example 11 | BiN-3-AL | A | A | A | A |
| Example 12 | BiN-4 | A | A | A | A |
| Example 13 | BiN-1-Ac | A | B | A | A |
| Example 14 | BiN-1-Ea | A | B | A | A |
| Example 15 | BiN-1-Ua | A | B | A | A |
| Example 16 | BiN-1-E | A | B | A | A |
| Example 17 | BiN-1-PX | A | A | A | A |
| Example 18 | BiN-1-PE | A | A | A | A |
| Example 19 | BiN-1-G | A | A | A | A |
| Example 20 | BiN-1-GE | A | A | A | A |
| Example 21 | BiN-1-SX | A | A | A | A |
| Example 22 | BiN-1-SE | A | A | A | A |
| Example 23 | BiN-1-Pr | A | A | A | A |
| Comparative Example 1 | CR-1 | A | C | C | A |

As shown in the table, all of the compositions using the compounds of the examples were able to provide a cured product in which all of refractive index, transparency, and film heat resistance are achieved at high dimensions. The composition used in the examples was excellent in solubility in solvents. On the other hand, the compound of Comparative Example 1 had a low refractive index and poor transparency.

As mentioned above, the present invention is not limited to the above embodiments and examples, and changes or modifications can be arbitrarily made without departing from the spirit of the present invention.

The composition for forming an optical component using the compound and the resin of the present embodiment has high refractive index and has high heat resistance attributed to its rigid structure, in spite of its relatively low molecular weight, and can therefore be used even under high temperature baking conditions. Also, the composition for forming an optical component of the present embodiment has high solubility in a safe solvent, suppressed crystallinity, good heat resistance and etching resistance, and provides a good optical component shape. Also, the composition is relatively prevented from being stained by heat treatment in a wide range from a low temperature to a high temperature. Therefore, the composition is useful as composition for forming various optical components.

Accordingly, the present invention is used in for example, electrical insulating materials, resins for resists, encapsulation resins for semiconductors, adhesives for printed circuit boards, electrical laminates mounted in electric equipment, electronic equipment, industrial equipment, and the like, matrix resins of prepregs mounted in electric equipment, electronic equipment, industrial equipment, and the like, buildup laminate materials, resins for fiber-reinforced plastics, resins for encapsulation of liquid crystal display panels, coating materials, various coating agents, adhesives, coating agents for semiconductors, resins for resists for semiconductors, and resins for underlayer film formation, requiring optical characteristics such as a high refractive index and high transparency, in the form of a film or a sheet, and additionally, can be used widely and effectively in optical components such as plastic lenses (prism lenses, lenticular lenses, microlenses, Fresnel lenses, viewing angle control lenses, contrast improving lenses, etc.), phase difference films, films for electromagnetic wave shielding, prisms, optical fibers, solder resists for flexible printed wiring, plating resists, interlayer insulating films for multilayer printed circuit boards, and photosensitive optical waveguides.

The disclosure of Japanese Patent Application No. 2018-144516 filed on July 31, 2018 is incorporated herein by reference in its entirety.

All literatures, patent applications, and technical standards described herein are incorporated herein by reference to the same extent as if each individual literature, patent application, or technical standard is specifically and individually indicated to be incorporated by reference.

## Claims

1. A composition for forming an optical component, comprising a polyphenol compound (B) and a solvent,
wherein the polyphenol compound (B) is at least one selected from a compound represented by the following formula (1) and a resin having a structure represented by the following formula (2): 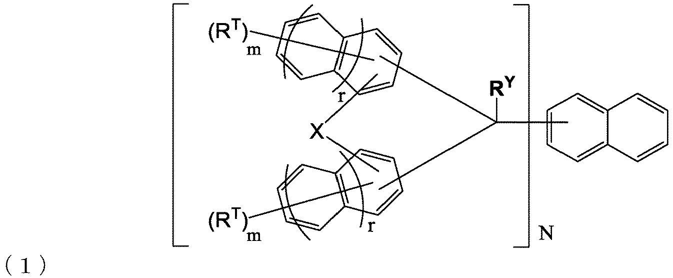 wherein
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2, and 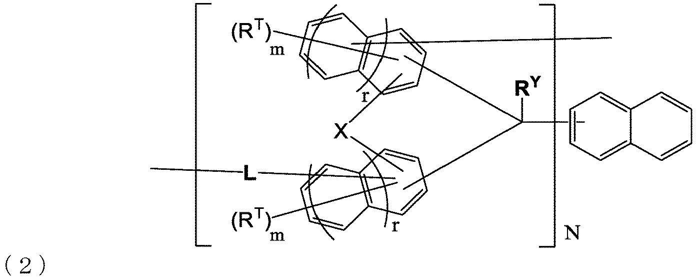
wherein L is an alkylene group having 1 to 30 carbon atoms and optionally having a substituent, an arylene group having 6 to 40 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2.

2. The composition for forming an optical component according to claim 1, wherein the polyphenol compound (B) is at least one selected from a compound represented by the following formula (1A) and a resin having a structure represented by the following formula (2A): 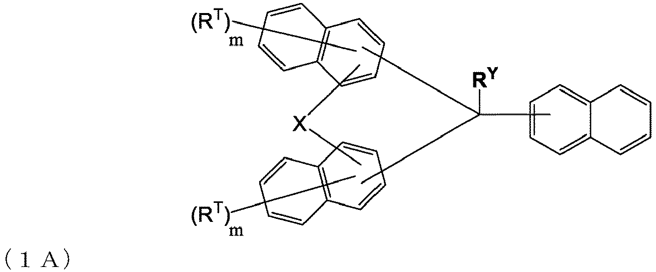 wherein R^{Y}, R^{T}, X, and m are as defined in the formula (1), and 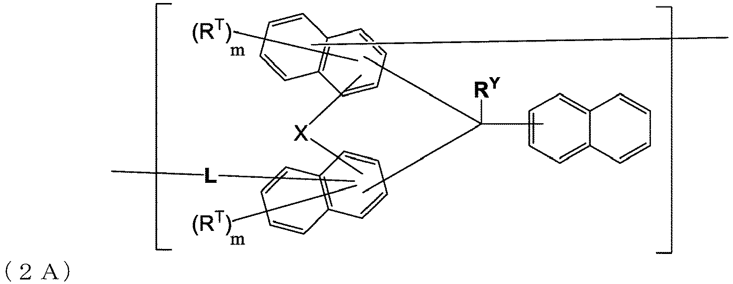 wherein L, R^{Y}, R^{T}, X, and m are as defined in the formula (2) .

3. The composition for forming an optical component according to claim 1, wherein the polyphenol compound (B) is at least one selected from a compound represented by the following formula (1B) or (1C) and a resin having a structure represented by the following formula (2B) or (2C) : 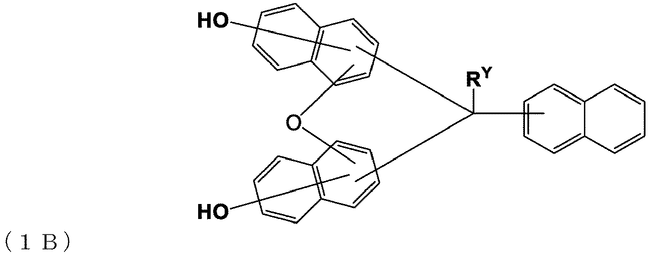 wherein R^{Y} is as defined in the formula (1), 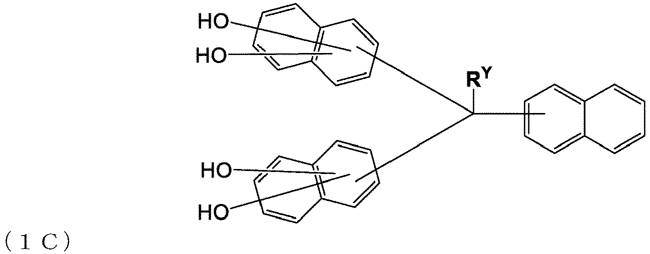 wherein R^{Y} is as defined in the formula (1), 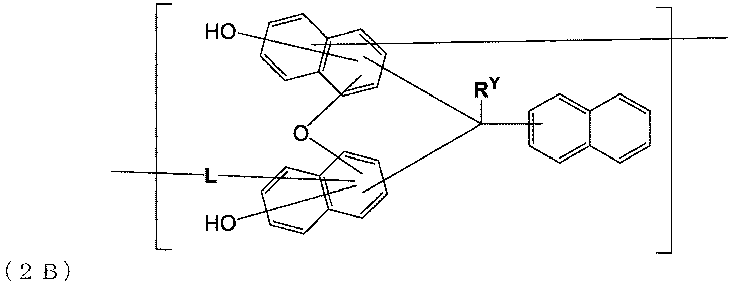 wherein L and R^{Y} are as defined in the formula (2), and 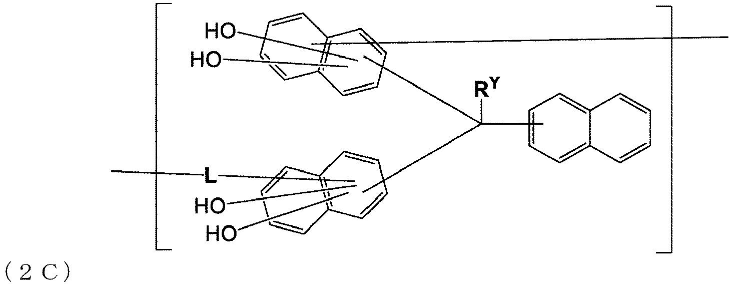 wherein L and R^{Y} are as defined in the formula (2).

4. The composition for forming an optical component according to any one of claims 1 to 3, further comprising an acid generating agent.

5. The composition for forming an optical component according to any one of claims 1 to 4, further comprising a crosslinking agent.

6. A compound represented by the following formula (1): 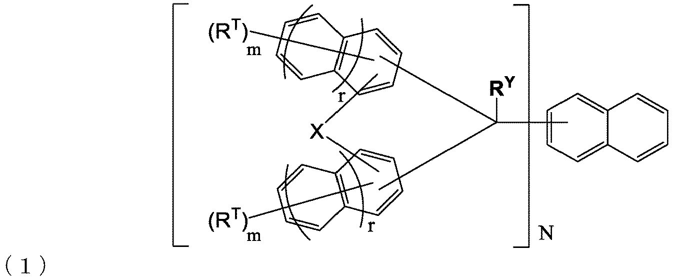 wherein
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2.

7. A resin having a structure represented by the following formula (2): 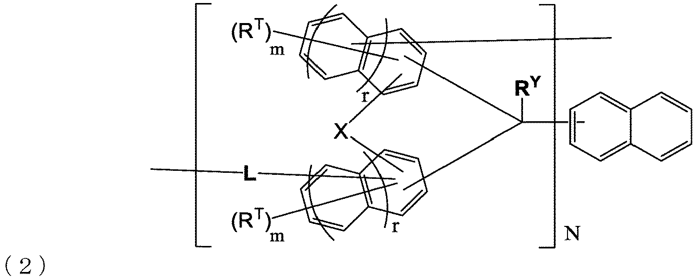
wherein L is an alkylene group having 1 to 30 carbon atoms and optionally having a substituent, an arylene group having 6 to 40 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 40 carbon atoms and optionally having a substituent;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a crosslinking group, a dissociation group, a thiol group, or a hydroxy group, wherein the alkyl group, the alkenyl group, the alkynyl group and the aryl group each optionally contain an ether bond, a ketone bond or an ester bond and at least one R^{T} is a crosslinking group, a dissociation group, a thiol group, or a hydroxy group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 1 to 2.

8. The compound according to claim 6, wherein the compound is represented by the following formula (1A): 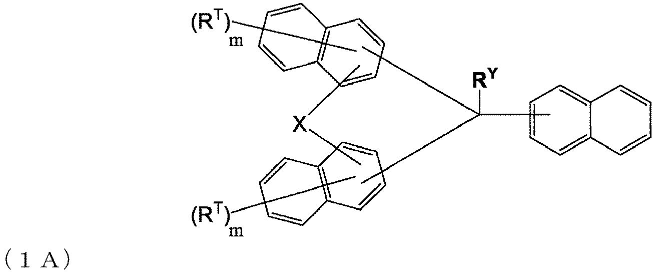 wherein R^{Y}, R^{T}, X, and m are as defined in the formula (1) .

9. The resin according to claim 7, wherein the resin has a structure represented by the following formula (2A) : 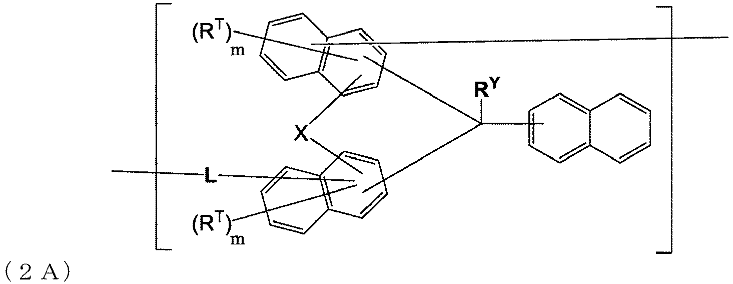 wherein L, R^{Y}, R^{T}, X, and m are as defined in the formula (2) .

10. The compound according to claim 6, wherein the compound is represented by the following formula (1B) or (1C) : 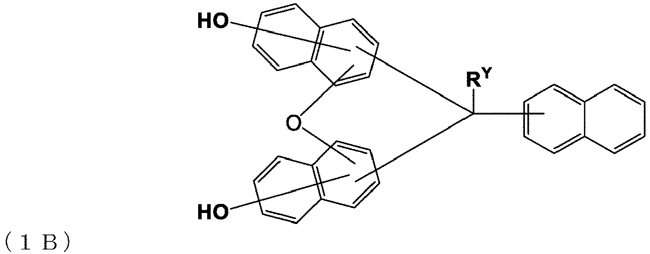 wherein R^{Y} is as defined in the formula (1), and 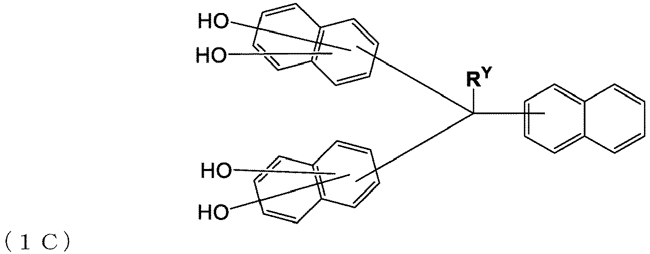 wherein R^{Y} is as defined in the formula (1).

11. The resin according to claim 7, wherein the resin has a structure represented by the following formula (2B) or (2C) : 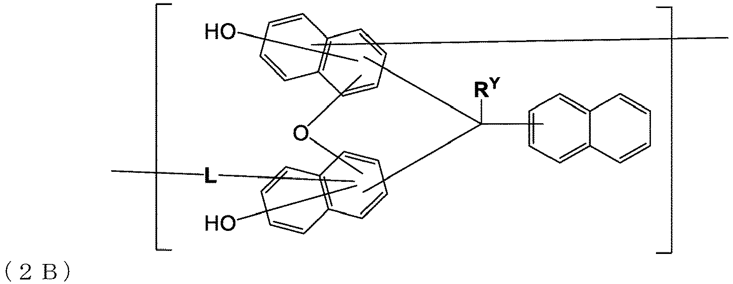 wherein L and R^{Y} are as defined in the formula (2), and 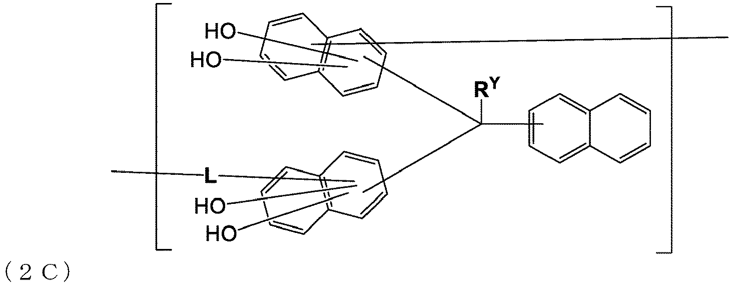 wherein L and R^{Y} are as defined in the formula (2).

12. A method for producing the compound according to claim 6, comprising the steps of: replacing a hydrogen atom represented by R^{Y-0} of a compound represented by the following formula (1-0) with a hydroxy group; and replacing the hydroxy group with R^{Y-1} of a compound represented by the following formula (1-1): 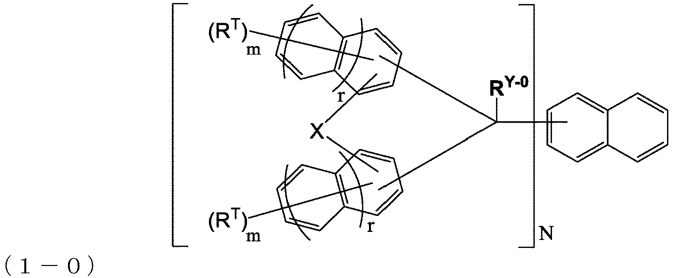 wherein
R^{Y-0} is a hydrogen atom; and
R^{T}, X, m, N, and r are as defined in the formula (1), and 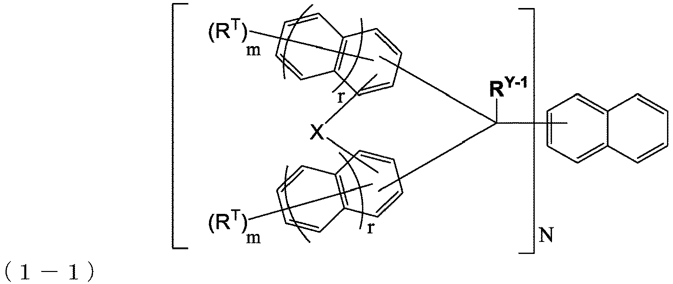
wherein
R^{Y-1} is an alkyl group having 1 to 30 carbon atoms and optionally having a substituent, or an aryl group having 6 to 40 carbon atoms and optionally having a substituent;
R^{T}, X, m, N, and r are as defined in the formula (1) .

13. An optical component obtained from the composition for forming an optical component according to any one of claims 1 to 5.
